(19) Europäisches Patentamt
European Patent Office
Office européen des brevets

(11) **EP 4 744 686 A1**

(12) **EUROPEAN PATENT APPLICATION**
published in accordance with Art. 153(4) EPC

(43) Date of publication:
**20.05.2026 Bulletin 2026/21**

(21) Application number: **24838819.1**

(22) Date of filing: **10.07.2024**

(51) International Patent Classification (IPC):
***A61L 27/34*** *(2006.01)* ***A61L 27/50*** *(2006.01)*
***A61L 31/14*** *(2006.01)* ***A61L 31/10*** *(2006.01)*
***C08F 292/00*** *(2006.01)*

(52) Cooperative Patent Classification (CPC):
**A61L 27/34; A61L 27/50; A61L 31/10; A61L 31/14; C08F 292/00**

(86) International application number:
**PCT/CN2024/104623**

(87) International publication number:
**WO 2025/011567 (16.01.2025 Gazette 2025/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.07.2023 CN 202310843194**
**08.04.2024 CN 202410417963**

(71) Applicant: **Shanghai Shashuo Co., Ltd.**
**Shanghai 201319 (CN)**

(72) Inventors:
- **YUAN, Zhishan**
**Shanghai 201319 (CN)**
- **XIONG, Xianzhong**
**Shanghai 201319 (CN)**
- **MOU, Tinghai**
**Shanghai 201319 (CN)**

(74) Representative: **Potter Clarkson**
**Chapel Quarter**
**Mount Street**
**Nottingham NG1 6HQ (GB)**

(54) **ZWITTERIONIC POLYMER AND USE THEREOF**

(57) The present disclosure relates to a zwitterionic polymer and the use thereof. Specifically, the present disclosure relates to a zwitterionic polymer, the use thereof as a biocompatible implantable material coating, and a method for preparing the coating. The coating can be used for inhibiting platelet aggregation and platelet adhesiveness caused by the exposure of blood to the surface of a material.

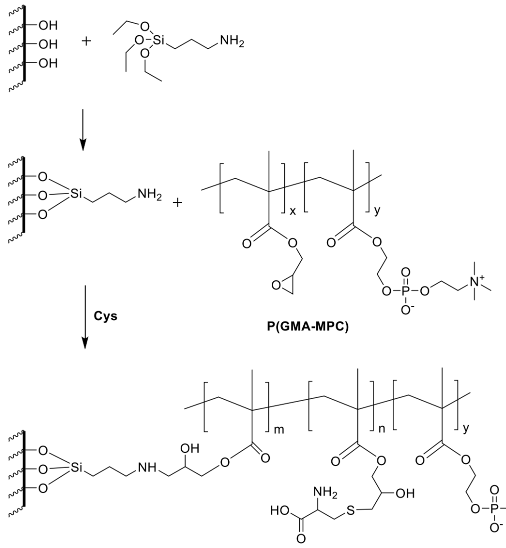


FIG. 1

EP 4 744 686 A1

## Description

### Technical Field

The present disclosure belongs to the field of medicine, and relates to a zwitterionic polymer and the use thereof as a biocompatible implantable material coating, wherein the coating can be used for inhibiting platelet aggregation and platelet adhesiveness caused by the exposure of blood to the surface of a material.

### Background Art

The use of implantable materials often requires contact with human blood or bodily fluid tissues, such as vascular grafts, catheters, stents, insulating materials for pacemaker and defibrillator leads, as well as extracorporeal bypass circuits and oxygenators. If these implantable materials do not possess good biocompatibility, direct contact with bodily fluids or blood will lead to protein aggregation or thrombus formation. This, in turn, will result in the compromised performance of the medical device and trigger severe complications in patients. For example, the placement of a vascular stent may damage the endothelium and induce an inflammatory response. To address the aforementioned issues, zwitterionic polymer materials are typically grafted onto the surface of implantable materials to form a coating, thereby improving the biocompatibility of the implantable materials.

Polymers containing the monomer 2-methacryloyloxyethyl phosphorylcholine (MPC) are extensively used in the field of implantable materials as a classic type of zwitterionic polymeric materials. Because such polymers can form a bimolecular structure similar to a biological membrane, they can effectively reduce the adsorption of fibrin and the adhesion and activation of platelets, thereby significantly improving the blood compatibility of the materials.

Patent application WO 2001007097A1 discloses the polymeric compound p-(GMA-MPC) copolymerised from two monomers, MPC and glycidyl methacrylate (GMA). It has good biocompatibility and biodegradability, and can be grafted onto the surface of medical devices through a ring-opening reaction with oxiranyl, thereby forming a coating with surface lubricity and blood compatibility. However, in recent years, it has been found that due to incomplete grafting of the polymer p-(GMA-MPC) during the grafting process, this will result in residual free oxiranyl on the surface of the polymer-modified materials. Since oxiranyl is cytotoxic, materials with coatings containing oxiranyl groups, if implanted into the human body, will cause significant harm to the human body.

Therefore, developing materials with better blood compatibility is a goal pursued by those skilled in the art.

### Summary of the Invention

The present disclosure provides a material, comprising a substrate and a coating, wherein the coating comprises a polymer, and the polymer comprises a structural unit having a group represented by formula 1-a, a structural unit having a group represented by formula 1-b, and a structural unit having a group represented by formula 1-c,

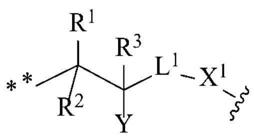
1-a ,
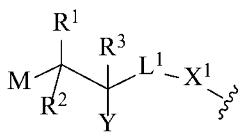
1-b and
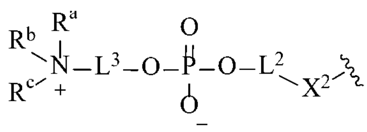
1-c ,

wherein:

- ** represents an end connected to the surface of the substrate, and the surface of the substrate is optionally treated with a modifier;
- $X^1$ and $X^2$ are the same or different, and are each independently selected from -O-, -S- or -N($R^d$)-;
- Y is selected from -OH, -SH or -N($R^eR^f$);
- $R^1$, $R^2$ and $R^3$ are the same or different, and are each independently selected from hydrogen, halogen, hydroxyl, mercapto, nitro, carboxyl, amino, cyano, $C_{1-6}$ alkyl or $C_{1-6}$ alkoxy, and the $C_{1-6}$ alkyl and $C_{1-6}$ alkoxy are optionally substituted with one or more groups selected from halogen, hydroxyl, mercapto, nitro, carboxyl, amino, cyano, $C_{1-6}$ alkyl and $C_{1-6}$ alkoxy,
- alternatively, $R^1$ and $R^2$, together with the atom to which they are connected, form an oxo group, a thio group, $C_{3-10}$ cycloalkyl, or 3- to 10-membered heterocycloalkyl;
- $L^1$ and $L^3$ are the same or different, and are each independently selected from a bond, alkylene or heteroalkylene, and the alkylene and heteroalkylene are optionally substituted with one or more groups selected from $C_{1-6}$ alkyl, $C_{1-6}$

haloalkyl, $C_{1-6}$ hydroxyalkyl, $C_{1-6}$ aminoalkyl, $C_{1-6}$ alkoxy, $C_{1-6}$ haloalkoxy, $C_{1-6}$ hydroxyalkoxy, $C_{1-6}$ aminoalkoxy, halogen, amino, hydroxyl, nitro, cyano, mercapto, carboxyl, an oxo group, a thio group or sulphonyl;
$L^2$ is selected from alkylene or heteroalkylene, and the alkylene and heteroalkylene are optionally substituted with one or more groups selected from $C_{1-6}$ alkyl, $C_{1-6}$ haloalkyl, $C_{1-6}$ hydroxyalkyl, $C_{1-6}$ aminoalkyl, $C_{1-6}$ alkoxy, $C_{1-6}$ haloalkoxy, $C_{1-6}$ hydroxyalkoxy, $C_{1-6}$ aminoalkoxy, halogen, amino, hydroxyl, nitro, cyano, mercapto, carboxyl, an oxo group, a thio group or sulphonyl;
M is a residue of amino acid (A), and the M is optionally substituted with one or more groups selected from halogen, amino, hydroxyl, nitro, cyano, amino, mercapto, carboxyl, $C_{1-6}$ alkyl or $C_{1-6}$ alkoxy;
$R^a$, $R^b$, $R^c$, $R^d$, $R^e$ and $R^f$ are the same or different, and are each independently selected from hydrogen, halogen, hydroxyl, $C_{1-6}$ alkyl or $C_{1-6}$ alkoxy, and the $C_{1-6}$ alkyl or $C_{1-6}$ alkoxy is optionally substituted with one or more groups selected from halogen, hydroxyl, cyano, nitro, $C_{1-6}$ alkyl, $C_{1-6}$ alkoxy and $C_{3-7}$ cycloalkyl.

In some embodiments, the polymer comprises a structural unit represented by formula I-a, a structural unit represented by formula I-b, and a structural unit represented by formula I-c,

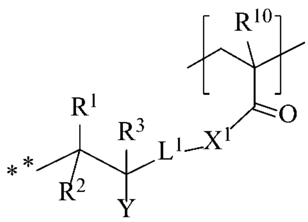
I-a

,

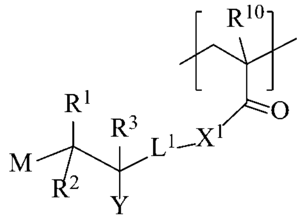
I-b

and

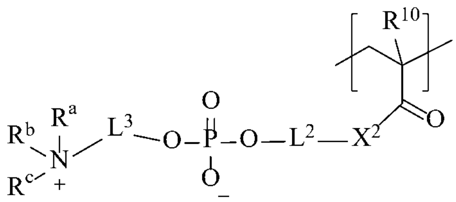
I-c

,

wherein:

** represents an end connected to the surface of the substrate;
each $R^{10}$ is independently selected from hydrogen, halogen, hydroxyl, mercapto, nitro, carboxyl, amino, cyano, $C_{1-6}$ alkyl or $C_{1-6}$ alkoxy, and the $C_{1-6}$ alkyl and $C_{1-6}$ alkoxy are optionally substituted with one or more groups selected from halogen, hydroxyl, mercapto, nitro, carboxyl, amino, cyano, $C_{1-6}$ alkyl or $C_{1-6}$ alkoxy;
$L^1$, $L^2$, $L^3$, $R^1$, $R^2$, $R^3$, M, Y, $X^1$, $X^2$, $R^a$, $R^b$, and $R^c$ are as defined above.

In certain embodiments, the polymer has a weight average molecular weight of 10,000 to 500,000 Da.
In certain embodiments, the polymer comprises a structural unit represented by formula I -A, a structural unit represented by formula I -B, and a structural unit represented by formula I -C,

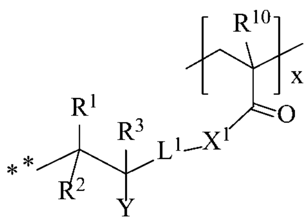
I-A

,

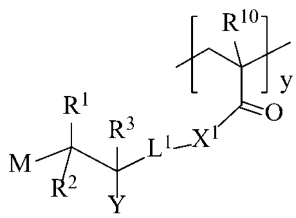
I-B

and

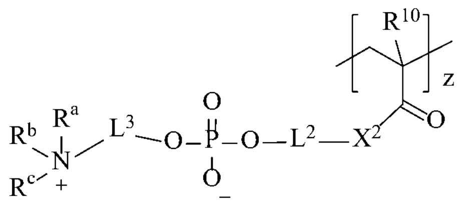

I-C

,

wherein, on a molar ratio basis, z : (x + y) is selected from 20 : 80 to 90 : 10, and $R^{10}$, $L^1$, $L^2$, $L^3$, $R^1$, $R^2$, $R^3$, M, Y, $X^1$, $X^2$, $R^a$, $R^b$, and $R^c$ are as defined above.

In certain embodiments, on a molar ratio basis, the z : (x+y) is selected from 20 : 80, 25 : 75, 30 : 70, 35 : 65, 40 : 60, 45 : 55, 50 : 50, 55 : 45, 60 : 40, 65 : 35, 70 : 30, 75 : 25, or 80 : 20. In certain embodiments, on a molar ratio basis, the z :(x+y) is selected from 40 : 60, 45 : 55, 50 : 50, 55 : 45, 60 : 40, 65 : 35, or 70 : 30.

In some embodiments, the Y is selected from -OH or -SH. In certain embodiments, the Y is -OH.

In some embodiments, the $L^1$ and $L^3$ are each independently selected from a bond or $C_{1-6}$ alkylene, and the $C_{1-6}$ alkylene is optionally substituted with one or more groups selected from $C_{1-6}$ alkyl, $C_{1-6}$ haloalkyl, $C_{1-6}$ hydroxyalkyl, $C_{1-6}$ aminoalkyl, halogen, amino, hydroxyl, cyano or mercapto.

In some embodiments, the $L^2$ is $C_{1-6}$ alkylene, which is optionally substituted with one or more groups selected from $C_{1-6}$ alkyl, $C_{1-6}$ haloalkyl, $C_{1-6}$ hydroxyalkyl, $C_{1-6}$ aminoalkyl, halogen, amino, hydroxyl, cyano or mercapto.

In some embodiments, the polymer comprises a structural unit having a group represented by formula 2-a, a structural unit having a group represented by formula 2-b, and a structural unit having a group represented by formula 2-c,

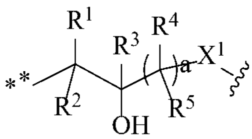

2-a

,

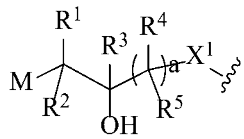

2-b

and

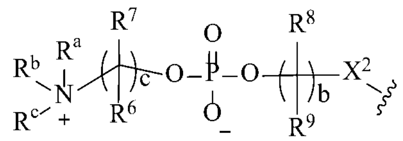

2-c

,

wherein: ** represents an end connected to the surface of the substrate;

$R^4$, $R^5$, $R^6$, $R^7$, $R^8$ and $R^9$ are the same or different, and are each independently selected from hydrogen, halogen, hydroxyl, mercapto, nitro, carboxyl, amino, cyano, $C_{1-6}$ alkyl, $C_{1-6}$ alkoxy, $C_{3-7}$ cycloalkyl or 3- to 7-membered heterocycloalkyl, and the $C_{1-6}$ alkyl, $C_{1-6}$ alkoxy, cycloalkyl or heterocycloalkyl is optionally substituted with one or more groups selected from halogen, hydroxyl, mercapto, nitro, carboxyl, amino, cyano, $C_{1-6}$ alkyl or $C_{1-6}$ alkoxy, alternatively, $R^4$ and $R^5$, $R^6$ and $R^7$, or $R^8$ and $R^9$, together with the atom to which they are connected, form an oxo group, a thio group, $C_{3-10}$ cycloalkyl, or 3- to 10-membered heterocycloalkyl;

a is selected from 0, 1, 2, 3, 4, 5, or 6;

b is selected from 1, 2, 3, 4, 5, or 6;

c is selected from 0, 1, 2, 3, 4, 5, or 6;

$X^1$, $X^2$, $R^1$, $R^2$, $R^3$, M, $R^a$, $R^b$, and $R^c$ are as defined above.

In some embodiments, the polymer comprises a structural unit represented by formula II -a, a structural unit represented by formula II -b, and a structural unit represented by formula II -c,

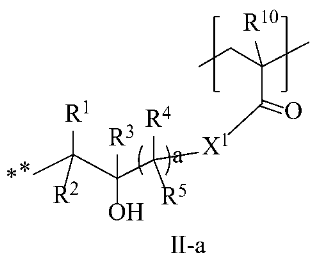

II-a

,

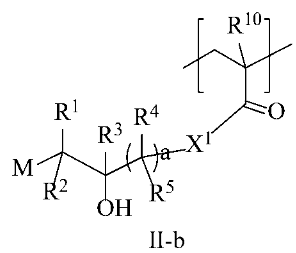

II-b

and

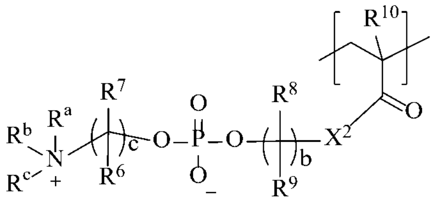

II-c

,

wherein:

** represents an end connected to the surface of the substrate;
$X^1$, $X^2$, $R^1$, $R^2$, $R^3$, $R^4$, $R^5$, $R^6$, $R^7$, $R^8$, $R^9$, $R^{10}$, M, $R^a$, $R^b$, $R^c$, a, b, and c are as defined above.

In certain embodiments, the polymer comprises a structural unit represented by formula II -A, a structural unit represented by formula II -B, and a structural unit represented by formula II -C,

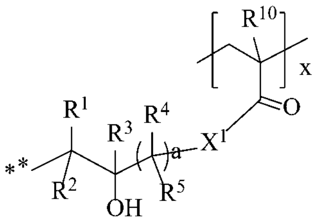

II-A

,

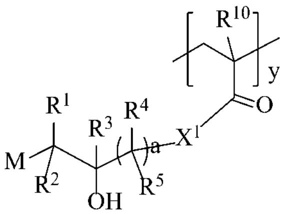

II-B

and

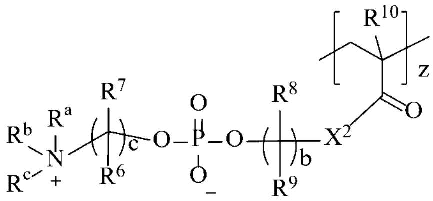

II-C

,

wherein:

** represents an end connected to the surface of the substrate;
$X^1$, $X^2$, $R^1$, $R^2$, $R^3$, $R^4$, $R^5$, $R^6$, $R^7$, $R^8$, $R^9$, $R^{10}$, M, $R^a$, $R^b$, $R^c$, a, b, c, x, y, and z are as defined above.

In some embodiments, the $X^1$ and $X^2$ are each independently selected from -O-or -N($R^d$)-, preferably -O-, and $R^d$ is as defined above.

In some embodiments, the polymer comprises a structural unit having a group represented by formula 3-a, a structural unit having a group represented by formula 3-b, and a structural unit having a group represented by formula 3-c,

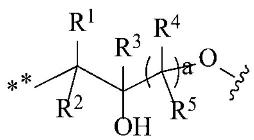

3-a

,

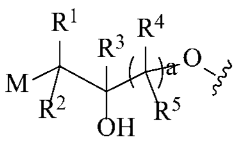

3-b

and

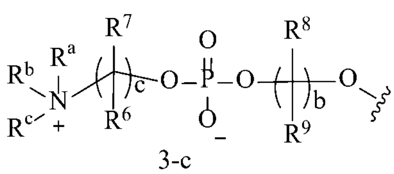

3-c

,

wherein: ** represents an end connected to the surface of the substrate;
$R^1$, $R^2$, $R^3$, $R^4$, $R^5$, $R^6$, $R^7$, $R^8$, $R^9$, M, $R^a$, $R^b$, $R^c$, a, b, and c are as defined above.

In some embodiments, the polymer comprises a structural unit represented by formula III-a, a structural unit represented by formula III-b, and a structural unit represented by formula III-c,

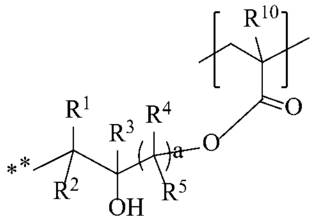

III-a

,

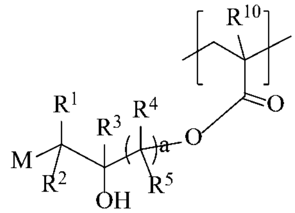

III-b

and

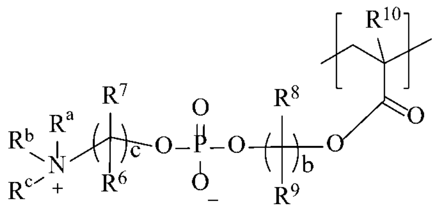

III-c

,

wherein: ** represents an end connected to the surface of the substrate;
$R^1$, $R^2$, $R^3$, $R^4$, $R^5$, $R^6$, $R^7$, $R^8$, $R^9$, $R^{10}$, M, $R^a$, $R^b$, $R^c$, a, b, and c are as defined above.

In certain embodiments, the polymer comprises a structural unit represented by formula III-A, a structural unit represented by formula III-B, and a structural unit represented by formula III-C,

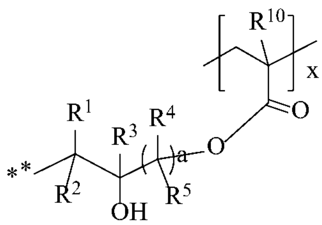

III-A

,

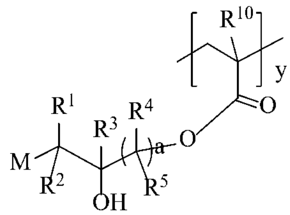

III-B

and

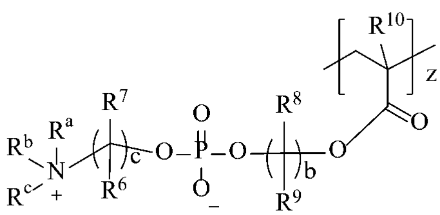

III-C

,

wherein: ** represents an end connected to the surface of the substrate;
$R^1$, $R^2$, $R^3$, $R^4$, $R^5$, $R^6$, $R^7$, $R^8$, $R^9$, $R^{10}$, M, $R^a$, $R^b$, $R^c$, a, b, c, x, y, and z are as defined above.

In some embodiments, the $R^1$, $R^2$, $R^3$, $R^4$, $R^5$, $R^6$, $R^7$, $R^8$ and $R^9$ are each independently selected from hydrogen or $C_{1-6}$ alkyl, and the $C_{1-6}$ alkyl is optionally substituted with one or more groups selected from halogen, hydroxyl, mercapto, nitro, carboxyl, amino, cyano, $C_{1-6}$ alkyl or $C_{1-6}$ alkoxy. In certain embodiments, $R^1$, $R^2$, $R^3$, $R^4$, $R^5$, $R^6$, $R^7$, $R^8$ and $R^9$ are each independently selected from hydrogen, methyl, ethyl, propyl, butyl, isopropyl or isobutyl.

In certain embodiments, the $R^{10}$ is $C_{1-6}$ alkyl, and the $C_{1-6}$ alkyl is optionally substituted with one or more groups selected from halogen, hydroxyl, mercapto, nitro, carboxyl, amino, cyano, $C_{1-6}$ alkyl or $C_{1-6}$ alkoxy. In certain embodiments, the $R^{10}$ is selected from $C_{1-6}$ haloalkyl or $C_{1-6}$ alkyl. In certain embodiments, the $R^{10}$ is selected from methyl, fluoromethyl, bromomethyl or iodomethyl.

In certain embodiments, the $R^a$, $R^b$ and $R^c$ are each independently selected from $C_{1-6}$ alkyl. In certain embodiments, the $R^a$, $R^b$ and $R^c$ are each independently selected from methyl, ethyl, propyl, butyl, isopropyl or isobutyl.

In some embodiments, the polymer comprises a structural unit having a group represented by formula 4-a, a structural unit having a group represented by formula 4-b, and a structural unit having a group represented by formula 4-c,

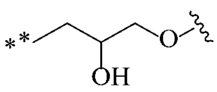
4-a ,
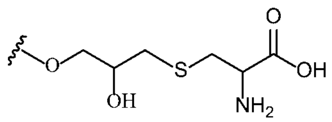
4-b and
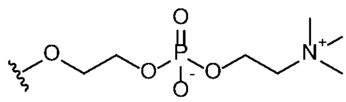
4-c ,

wherein: ** represents an end connected to the surface of the substrate.

In some embodiments, the amino acid (A) is selected from glycine, alanine, valine, isoleucine, leucine, methionine, phenylalanine, tryptophan, serine, threonine, cysteine, asparagine, glutamine, tyrosine, aspartic acid, glutamic acid, lysine, arginine, histidine or taurine. In some embodiments, the amino acid (A) is selected from glycine, phenylalanine, threonine, serine, histidine or cysteine.

In some embodiments, the amino acid (A) is selected from glycine, threonine, serine or cysteine.

In certain embodiments, the polymer comprises a structural unit represented by formula IV-a, a structural unit represented by formula |V-b, and a structural unit represented by formula IV-c,

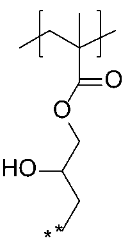
IV-a ,
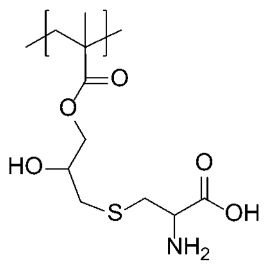
IV-b and
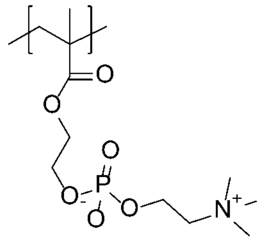
IV-c ,

wherein: ** represents an end connected to the surface of the substrate.

In certain embodiments, the polymer comprises a structural unit represented by formula IV-A, a structural unit represented by formula IV-B, and a structural unit represented by formula IV-C,

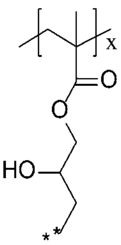
IV-A ,
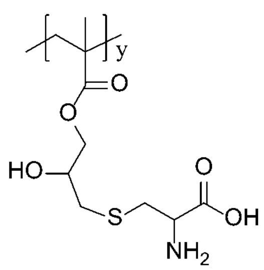
IV-B and
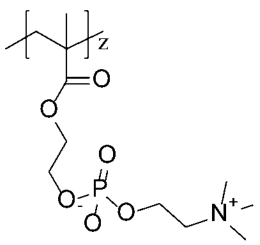
IV-C ,

wherein: ** represents an end connected to the surface of the substrate, and x, y and z are as defined above.

In certain embodiments, the amino acid (A) is connected to the C-terminus of formula 1-b, formula 2-b, formula 3-b, formula 4-b, formula I -b, formula II -b, formula III-b, IV-b, formula I -B, formula II -B, formula III-B or IV-B via its side chain

group, and the side chain group is selected from hydrogen, hydroxyl, carboxyl, amino or mercapto. In certain embodiments, the side chain group is selected from hydrogen, hydroxyl or mercapto. For example, in certain embodiments, the amino acid (A) is cysteine, which is connected to the C-terminus of formula 1-b, formula 2-b, formula 3-b, formula I -b, formula II -b, formula III-b, IV-b, formula I -B, formula II -B, formula III-B or IV-B via a side chain group, mercapto, wherein M is

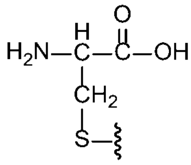

.

The various structural units of the polymer described in the present disclosure may include more than one arrangement and combination, but all the arrangements and combinations refer to the same polymer. Taking a polymer comprising structural units of formula IV-A, formula IV-B and formula IV-C as an example, the polymer may be represented by, but not limited to, formula A-1, formula A-2 or formula A-3, wherein formula A-1, formula A-2 and formula A-3 all represent the same polymer:

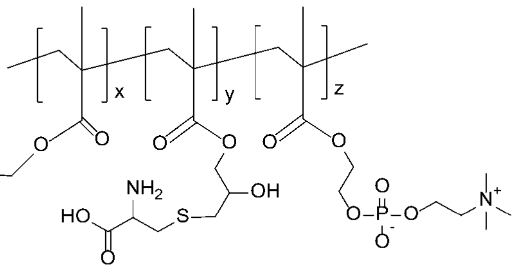

A-1

,

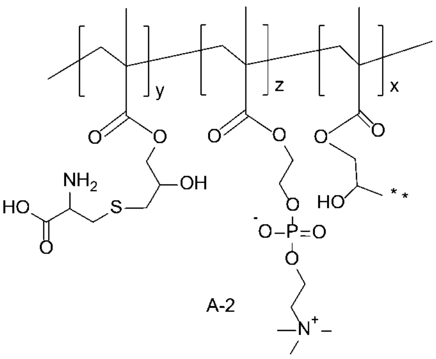

A-2

,

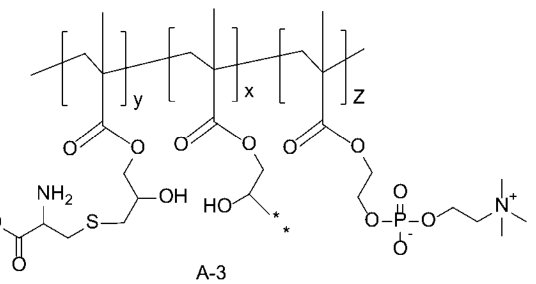

A-3

,

wherein x, y and z are as defined above.

The triblock polymers described in the present disclosure are all represented by the arrangement and combination order of the structural units shown in formula A-1. The abbreviations corresponding to formula A-1, formula A-2 or formula A-3 are all denoted as P(GMA-cysGMA-MPC).

In some embodiments, the polymer in the coating of the present disclosure is present in the form of a residue, which is covalently connected to the surface of the substrate via **, or covalently connected to the surface of the substrate treated with a modifier via **. For example, the polymer is present in the form of formula A-1, wherein ** represents an end connected to the surface of the substrate,

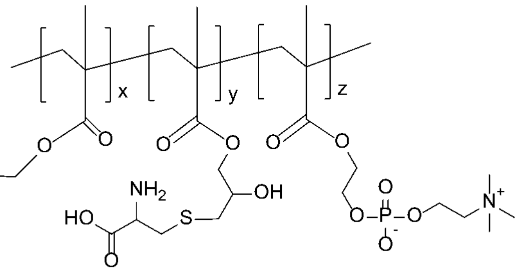

A-1

.

In certain embodiments, the aforementioned polymer is connected to the surface of the substrate via a bond. In certain embodiments, the aforementioned polymer is connected to the surface of the substrate via a modifier.

In some embodiments, the coating further comprises a silane layer containing the aforementioned modifier. In certain embodiments, the silane layer is an inner coating layer and is covalently connected to the surface of the substrate; and the polymer layer is an outer coating layer and is covalently connected to the silane layer.

In some embodiments, the modifier is a coupling agent represented by formula V,

$$(R^{11}-O)_3Si-R^{12}$$

V

,

wherein: $R^{11}$ is selected from $C_{1-6}$ alkyl, $C_{1-6}$ haloalkyl, $C_{1-6}$ hydroxyalkyl or $C_{1-6}$ aminoalkyl; $R^{12}$ is selected from $C_{1-6}$ hydroxyalkyl, $C_{1-6}$ aminoalkyl, $C_{1-6}$ carboxyalkyl or $C_{1-6}$ mercaptoalkyl, and the $C_{1-6}$ hydroxyalkyl, $C_{1-6}$ aminoalkyl, $C_{1-6}$ carboxyalkyl and $C_{1-6}$ mercaptoalkyl are optionally substituted with one or more groups selected from $C_{1-6}$ hydroxyalkyl, $C_{1-6}$ aminoalkyl, $C_{1-6}$ carboxyalkyl or $C_{1-6}$ mercaptoalkyl.

In some embodiments, the coupling agent is connected to the surface of the substrate via $R^{11}$ to form a silane layer, and the surface of the substrate is optionally hydroxylated. In certain embodiments, $R^{11}$ is $C_{1-6}$ alkyl. In certain embodiments, $R^{11}$ is selected from methyl, ethyl, propyl or butyl. In certain embodiments, $R^{11}$ is ethyl.

In some embodiments, the coupling agent is connected to the ** end of the aforementioned polymer via $R^{12}$ to form a polymer layer. Specifically, the coupling agent is connected to the ** end of formula 1-a, formula 2-a, formula 3-a, formula 4-a, formula I -a, formula II -a, formula III-a, formula IV-a, formula I -A, formula II -A, formula III-A or formula IV-A via a reactive group on $R^{12}$, and the reactive group is selected from hydroxyl, amino, carboxyl or mercapto. In certain embodiments, the reactive group is selected from amino, hydroxyl or mercapto. In certain embodiments, the reactive group is amino. For example, in certain embodiments, the coupling agent is connected to the ** end of the aforementioned polymer via the amino group of $R^{12}$ to form a polymer layer. In certain embodiments, the $R^{12}$ is $C_{1-6}$ aminoalkyl, and the $C_{1-6}$ aminoalkyl is optionally substituted with one or more groups selected from $C_{1-6}$ hydroxyalkyl, $C_{1-6}$ aminoalkyl, $C_{1-6}$ carboxyalkyl or $C_{1-6}$ mercaptoalkyl. In certain embodiments, the $R^{12}$ is selected from methylamino, ethylamino, propylamino or butylamino. In certain embodiments, the $R^{12}$ is propylamino.

In some embodiments, the coupling agent is selected from 3-aminopropyltrimethoxysilane (KH540), 3-aminopropyltriethoxysilane (KH550), 3-(2-aminoethyl)aminopropyltrimethoxysilane (KH792), 3-(2-aminoethyl)aminopropyltriethoxysilane (KH791), 3-(2-aminoethylamino)propyldimethoxymethylsilane (KH602), 3-hydroxypropyltriethoxysilane, 3-hydroxypropyltrimethoxysilane, 3-mercaptopropyltrimethoxysilane, 3-mercaptopropyltriethoxysilane, 4-(trimethoxysilyl)butyric acid, 4-(triethoxysilyl)butyric acid, bis(2-hydroxyethyl)aminopropyltriethoxysilane, bis(2-hydroxyethyl)aminopropyltrimethoxysilane, or 3-aminopropyltriisopropoxysilane. In certain embodiments, the coupling agent is selected from 3-aminopropyltrimethoxysilane (KH540) or 3-aminopropyltriethoxysilane (KH550). In certain embodiments, the coupling agent is 3-aminopropyltriethoxysilane (KH550).

In another aspect, the present disclosure further provides a material, comprising a substrate and a coating, wherein the coating is formed by reacting a polymer comprising a structural unit having a group represented by formula 1-d and a structural unit having a group represented by formula 1-c with the coupling agent as defined above, and then reacting with the amino acid (A) as defined above,

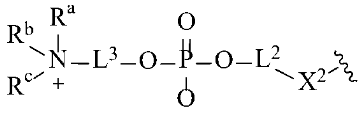

1-c

,

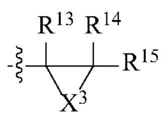

1-d

,

wherein:

$X^3$ is selected from -O-, -S- or -N($R^d$)-;

$R^{13}$, $R^{14}$ and $R^{15}$ are the same or different, and are each independently selected from hydrogen, halogen, hydroxyl, mercapto, nitro, carboxyl, amino, cyano, $C_{1-6}$ alkyl or $C_{1-6}$ alkoxy, and the $C_{1-6}$ alkyl and $C_{1-6}$ alkoxy are optionally substituted with one or more groups selected from halogen, hydroxyl, mercapto, nitro, carboxyl, amino, cyano, $C_{1-6}$ alkyl or $C_{1-6}$ alkoxy,

alternatively, $R^{14}$ and $R^{15}$, together with the atom to which they are connected, form an oxo group, a thio group, $C_{3-10}$ cycloalkyl, or 3- to 10-membered heterocycloalkyl;

each $R^d$ is independently selected from hydrogen, halogen, hydroxyl, $C_{1-6}$ alkyl or $C_{1-6}$ alkoxy, and the $C_{1-6}$ alkyl or $C_{1-6}$ alkoxy is optionally substituted with one or more groups selected from halogen, hydroxyl, cyano, nitro, $C_{1-6}$ alkyl, $C_{1-6}$ alkoxy or $C_{3-7}$ cycloalkyl.

In some embodiments, the polymer comprises a structural unit represented by formula I-d and a structural unit represented by formula I-c,

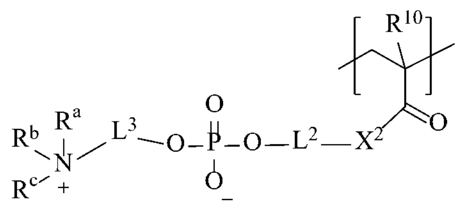

I-c ,

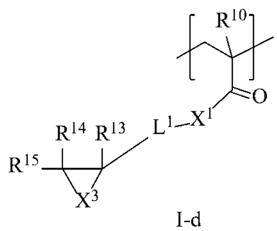

I-d ,

wherein: $R^{10}$, $L^1$, $L^2$, $L^3$, $X^1$, $X^3$, $R^{13}$, $R^{14}$, $R^{15}$, $R^a$, $R^b$, and $R^c$ are as defined above.

In certain embodiments, the polymer has a weight average molecular weight of 10,000 to 500,000 Da.

In certain embodiments, the polymer comprises a structural unit represented by formula I -D and a structural unit represented by formula I -C',

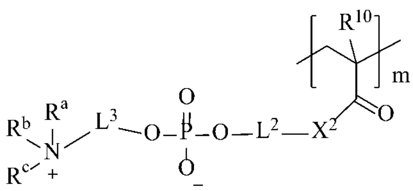

I-C' and

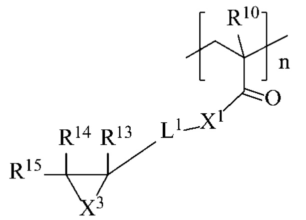

I-D ,

wherein:

on a molar ratio basis, m : n is selected from 20 : 80 to 90 : 10,

$R^a$, $R^b$, $R^c$, $L^2$, $L^3$, $R^{10}$, $L^1$, $X^1$, $X^2$, $R^{13}$, $R^{14}$, and $R^{15}$ are as defined above.

In certain embodiments, on a molar ratio basis, the m : n is selected from 20 : 80, 25 : 75, 30 : 70, 35 : 65, 40 : 60, 45 : 55, 50 : 50, 55 : 45, 60 : 40, 65 : 35, 70 : 30, 75 : 25, or 80 : 20. In certain embodiments, on a molar ratio basis, the m : n is selected from 40 : 60, 45 : 55, 50 : 50, 55 : 45, 60 : 40, 65 : 35, or 70 : 30.

In some embodiments, the $X^3$ is selected from -O- or -S-. In certain embodiments, $X^3$ is -O-.

In some embodiments, the group represented by formula 1-d is as represented by formula 2-d, and $R^{13}$, $R^{14}$ and $R^{15}$ are as defined above,

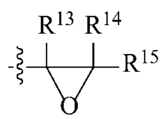

2-d .

In some embodiments, the polymer comprises a structural unit represented by formula II -d and a structural unit represented by formula II -c,

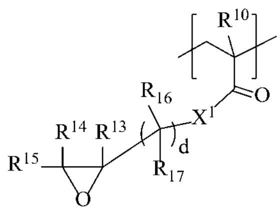

II-d ,

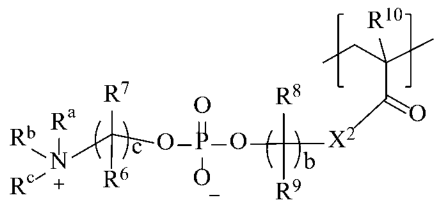

II-c ,

wherein:

$R^{16}$ and $R^{17}$ are the same or different, and are each independently selected from hydrogen, halogen, hydroxyl, mercapto, nitro, carboxyl, amino, cyano, $C_{1-6}$ alkyl or $C_{1-6}$ alkoxy, and the $C_{1-6}$ alkyl and $C_{1-6}$ alkoxy are optionally substituted with one or more groups selected from halogen, hydroxyl, mercapto, nitro, carboxyl, amino, cyano, $C_{1-6}$ alkyl or $C_{1-6}$ alkoxy,

alternatively, $R^{16}$ and $R^{17}$, together with the atom to which they are connected, form an oxo group, a thio group, $C_{3-10}$ cycloalkyl, or 3- to 10-membered heterocycloalkyl;

d is selected from 0, 1, 2, 3, 4, 5, or 6;

$R^6$, $R^7$, $R^8$, $R^9$, $R^{10}$, $X^1$, $R^{13}$, $R^{14}$, $R^{15}$, and b are as defined above.

In some embodiments, the polymer comprises a structural unit represented by formula II -D and a structural unit represented by formula II -C',

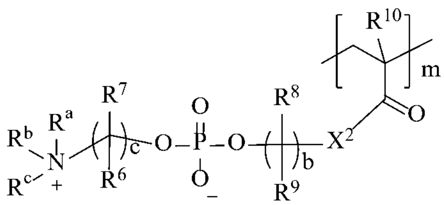

II-C' and

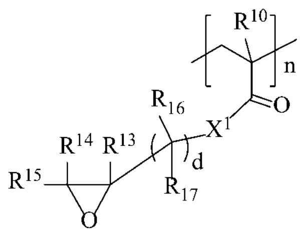

II-D ,

wherein: $X^1$, $X^2$, $R^{13}$, $R^{14}$, $R^{15}$, $R^{16}$, $R^{17}$, $R^6$, $R^7$, $R^8$, $R^9$, $R^{10}$, $R^a$, $R^b$, $R^c$, b, c, d, m, and n are as defined above.

In some embodiments, the $X^3$ is selected from -O- or -S-, preferably -O-.

In some embodiments, the polymer comprises a structural unit represented by formula III-d and a structural unit represented by formula III-c,

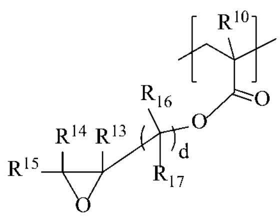

III-d ,

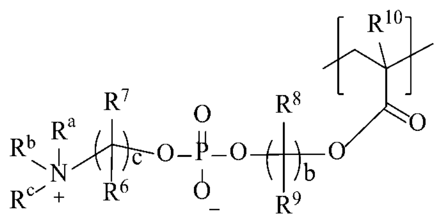

III-c ,

wherein d is selected from 0, 1, 2, 3, 4, 5, or 6,

$R^{13}$, $R^{14}$, $R^{15}$, $R^{16}$, $R^{17}$, $R^6$, $R^7$, $R^8$, $R^9$, $R^{10}$, $R^a$, $R^b$, $R^c$, b, c, and d are as defined above.

In some embodiments, the polymer comprises a structural unit represented by formula III-D and a structural unit represented by formula III-C',

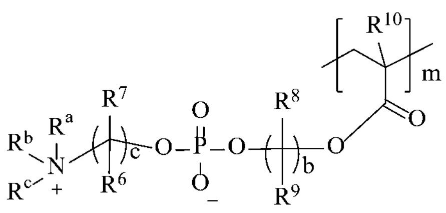

III-C'

and

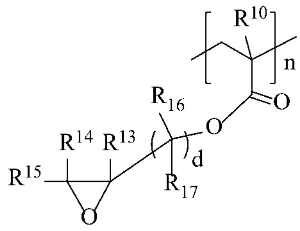

III-D ,

wherein: $R^{13}$, $R^{14}$, $R^{15}$, $R^{16}$, $R^{17}$, $R^6$, $R^7$, $R^8$, $R^9$, $R^{10}$, $R^a$, $R^b$, $R^c$, b, c, d, m, and n are as defined above.

In some embodiments, the $R^{13}$, $R^{14}$ and $R^{15}$ are each independently hydrogen.

In some embodiments, $R^{16}$ and $R^{17}$ are each independently selected from hydrogen or $C_{1-6}$ alkyl, and the $C_{1-6}$ alkyl is optionally substituted with one or more groups selected from halogen, hydroxyl, mercapto, nitro, carboxyl, amino, cyano, $C_{1-6}$ alkyl or $C_{1-6}$ alkoxy. In certain embodiments, $R^{16}$ and $R^{17}$ are each independently selected from hydrogen, methyl, ethyl, propyl, butyl, isopropyl or isobutyl.

In some embodiments, the group represented by formula 1-d is as represented by formula 3-d,

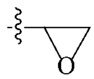

3-d .

In certain embodiments, the polymer comprises a structural unit represented by formula IV-c and a structural unit represented by formula IV-d,

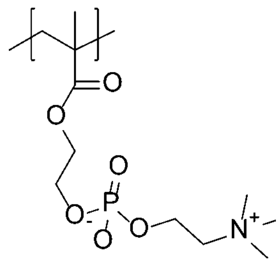

IV-c

and

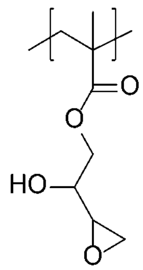

IV-d .

In certain embodiments, the polymer comprises a structural unit represented by formula IV-C' and a structural unit represented by formula IV-D,

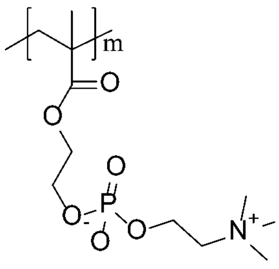

IV-C'

and

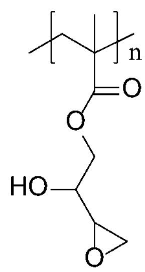

IV-D ,

wherein m and n are as defined above.

In some embodiments, the coupling agent reacts with the group represented by formula 1-d, formula 2-d or formula 3-d in the polymer via $R^{12}$. Specifically, the coupling agent undergoes a ring-opening reaction with the group represented by formula 1-d, formula 2-d or formula 3-d in the polymer via a reactive group on $R^{12}$, and the reactive group is selected from hydroxyl, amino, carboxyl or mercapto. In certain embodiments, the reactive group is selected from amino,

hydroxyl or mercapto. In certain embodiments, the reactive group is amino.

In another aspect, the present disclosure further provides a coating comprising the polymer layer as defined above, wherein the polymer layer comprises a polymer containing a structural unit having a group represented by formula 1-a, a structural unit having a group represented by formula 1-b, and a structural unit having a group represented by formula 1-c.

In some embodiments, the polymer comprises a structural unit having a group represented by formula 2-a, a structural unit having a group represented by formula 2-b, and a structural unit having a group represented by formula 2-c.

In some embodiments, the polymer comprises a structural unit having a group represented by formula 3-a, a structural unit having a group represented by formula 3-b, and a structural unit having a group represented by formula 3-c.

In some embodiments, the polymer comprises a structural unit having a group represented by formula 4-a, a structural unit having a group represented by formula 4-b, and a structural unit having a group represented by formula 4-c.

In some embodiments, the polymer comprises a structural unit represented by formula I-a, a structural unit represented by formula I-b, and a structural unit represented by formula I-c. In certain embodiments, the polymer comprises a structural unit represented by formula I -A, a structural unit represented by formula I -B, and a structural unit represented by formula I -C.

In some embodiments, the polymer comprises a structural unit represented by formula II -a, a structural unit represented by formula II -b, and a structural unit represented by formula II -c. In certain embodiments, the polymer comprises a structural unit represented by formula II -A, a structural unit represented by formula II -B, and a structural unit represented by formula II -C.

In some embodiments, the polymer comprises a structural unit represented by formula III-a, a structural unit represented by formula III-b, and a structural unit represented by formula III-c. In certain embodiments, the polymer comprises a structural unit represented by formula III-A, a structural unit represented by formula III-B, and a structural unit represented by formula III-C.

In some embodiments, the polymer comprises a structural unit represented by formula IV-a, a structural unit represented by formula IV-b, and a structural unit represented by formula IV-c. In certain embodiments, the polymer comprises a structural unit represented by formula IV-A, a structural unit represented by formula IV-B, and a structural unit represented by formula IV-C.

In some embodiments, the coating further comprises the silane layer as defined above. The silane layer comprises the coupling agent as defined above.

In another aspect, the present disclosure further provides a coating, wherein the coating is formed by reacting a polymer comprising a structural unit having a group represented by formula 1-d and a structural unit having a group represented by formula 1-c with the coupling agent as defined above, and then reacting with amino acid (A), and
the amino acid (A) is selected from glycine, alanine, valine, isoleucine, leucine, methionine, phenylalanine, tryptophan, serine, threonine, cysteine, asparagine, glutamine, tyrosine, aspartic acid, glutamic acid, lysine, arginine, histidine or taurine, preferably glycine, phenylalanine, threonine, serine, histidine or cysteine, most preferably glycine, threonine, serine or cysteine.

In some embodiments, the polymer comprises a structural unit having a group represented by formula 2-d and a structural unit having a group represented by formula 2-c.

In some embodiments, the polymer comprises a structural unit having a group represented by formula 3-d and a structural unit having a group represented by formula 3-c or formula 4-c.

In some embodiments, the polymer comprises a structural unit having a group represented by formula I -d and a structural unit having a group represented by formula I -c. In certain embodiments, the polymer comprises a structural unit represented by formula I -D and a structural unit represented by formula I -C'.

In some embodiments, the polymer comprises a structural unit having a group represented by formula II -d and a structural unit having a group represented by formula II -c. In certain embodiments, the polymer comprises a structural unit represented by formula II -D and a structural unit represented by formula II -C'.

In some embodiments, the polymer comprises a structural unit having a group represented by formula III-d and a structural unit having a group represented by formula III -c. In certain embodiments, the polymer comprises a structural unit represented by formula III-D and a structural unit represented by formula III-C'.

In some embodiments, the polymer comprises a structural unit having a group represented by formula IV-d and a structural unit having a group represented by formula IV-c. In certain embodiments, the polymer comprises a structural unit represented by formula IV-D and a structural unit represented by formula IV-C'.

In another aspect, the present disclosure further provides a method for preparing a coating, comprising: reacting a polymer comprising a structural unit having a group represented by formula 1-d and a structural unit having a group represented by formula 1-c with the coupling agent as defined above, and then reacting with amino acid (A), wherein
the amino acid (A) is selected from glycine, alanine, valine, isoleucine, leucine, methionine, phenylalanine, tryptophan, serine, threonine, cysteine, asparagine, glutamine, tyrosine, aspartic acid, glutamic acid, lysine, arginine, histidine or taurine, preferably glycine, phenylalanine, threonine, serine, histidine or cysteine, most preferably glycine, threonine,

serine or cysteine.

In some embodiments, the polymer comprises a structural unit having a group represented by formula 2-d and a structural unit having a group represented by formula 2-c.

In some embodiments, the polymer comprises a structural unit having a group represented by formula 3-d and a structural unit having a group represented by formula 3-c or formula 4-c.

In some embodiments, the polymer comprises a structural unit having a group represented by formula I -d and a structural unit having a group represented by formula I -c. In certain embodiments, the polymer comprises a structural unit represented by formula I -D and a structural unit represented by formula I -C'.

In some embodiments, the polymer comprises a structural unit having a group represented by formula II -d and a structural unit having a group represented by formula II -c. In certain embodiments, the polymer comprises a structural unit represented by formula II -D and a structural unit represented by formula II -C'.

In some embodiments, the polymer comprises a structural unit having a group represented by formula III-d and a structural unit having a group represented by formula III -c. In certain embodiments, the polymer comprises a structural unit represented by formula III-D and a structural unit represented by formula III-C'.

In some embodiments, the polymer comprises a structural unit having a group represented by formula IV-d and a structural unit having a group represented by formula IV-c. In certain embodiments, the polymer comprises a structural unit represented by formula IV-D and a structural unit represented by formula IV-C'.

In another aspect, the present disclosure further provides a method for preparing a biocompatible implantable material, comprising: connecting the aforementioned coupling agent to the surface of a substrate of a material via $R^{11}$ to form a silane layer; and reacting the coupling agent via $R^{12}$ with the group represented by formula 1-d, formula 2-d or formula 3-d in the aforementioned polymer to form a polymer layer, wherein the surface of the substrate is optionally hydroxylated. Specifically, the coupling agent undergoes a ring-opening reaction with the group represented by formula 1-d, formula 2-d or formula 3-d in the aforementioned polymer via a reactive group on $R^{12}$, and the reactive group is selected from hydroxyl, amino, carboxyl or mercapto. In certain embodiments, the reactive group is selected from amino, hydroxyl or mercapto. In certain embodiments, the reactive group is amino.

In another aspect, the present disclosure further provides a method for inhibiting platelet aggregation and platelet adhesiveness caused by the exposure of blood to the surface of a material, comprising using the aforementioned material.

In another aspect, the present disclosure further provides a method for inhibiting platelet aggregation and platelet adhesiveness caused by the exposure of blood to the surface of a material, comprising applying the aforementioned coating to the surface of a substrate of a material.

In another aspect, the present disclosure further provides a method for inhibiting platelet aggregation and platelet adhesiveness caused by the exposure of blood to the surface of a material, comprising applying the coating prepared by the aforementioned method to the surface of a substrate of a material.

In some embodiments, the structural unit represented by formula I-d or formula I-D is derived from a monomeric compound selected from:

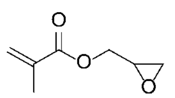, 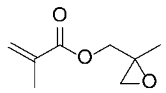, 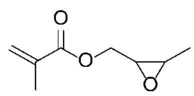, 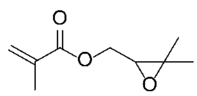,

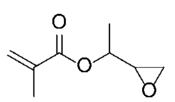, 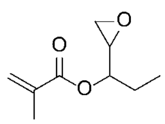, 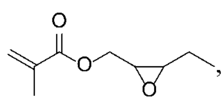, 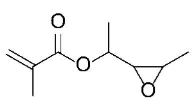,

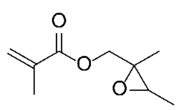, 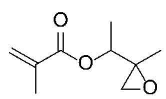, 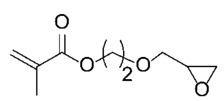,

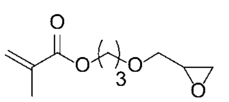, 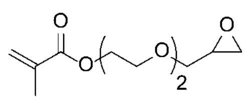, 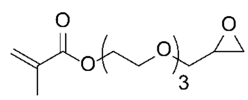,

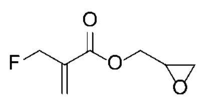, 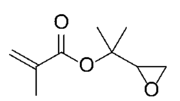, 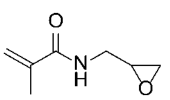, 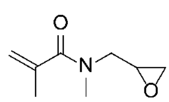,

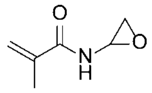 , 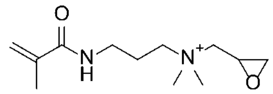 , 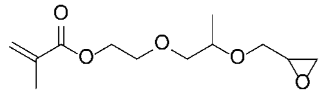 ,

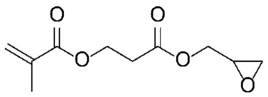 , 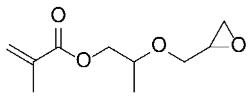 , 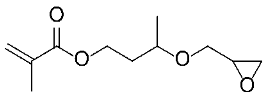 ,

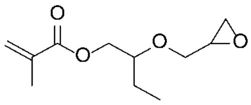 , 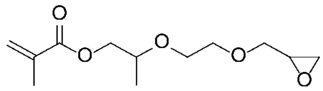 or 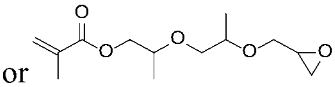 .

In some embodiments, the structural unit represented by formula II -d or formula II -D is derived from a monomeric compound selected from:

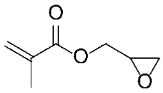 , 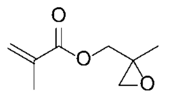 , 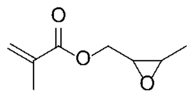 , 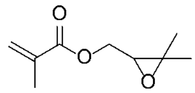 ,

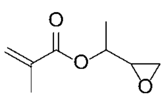 , 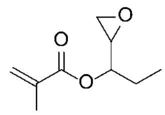 , 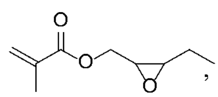 , 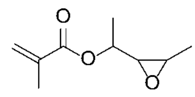 ,

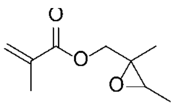 , 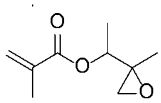 , 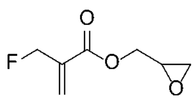 , 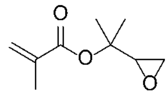 ,

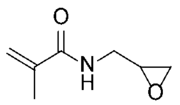 , 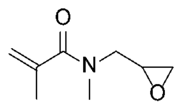 or 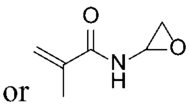 .

In some embodiments, the structural unit represented by formula III -d or formula III-D is derived from a monomeric compound selected from:

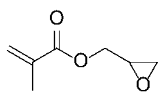 , 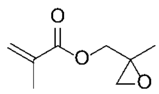 , 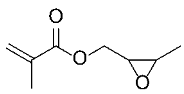 , 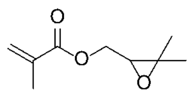 ,

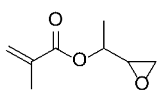 , 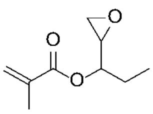 , 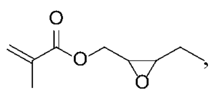 , 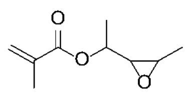 ,

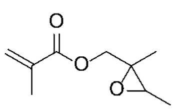 , 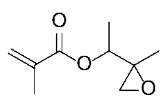 , 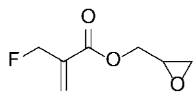 or 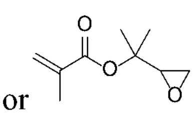 .

In some embodiments, the structural unit represented by formula I-b or formula I-B is derived from a monomeric compound selected from:

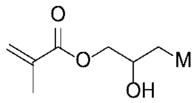 , 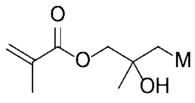 , 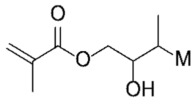 , 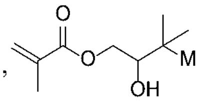 ,

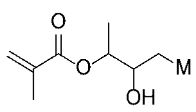 , 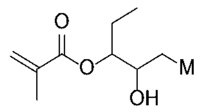 , 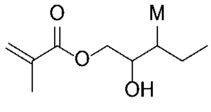 , 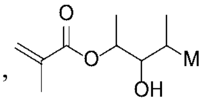 ,

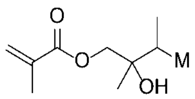 , 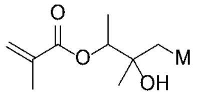 , 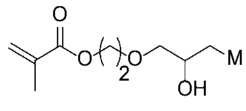 ,

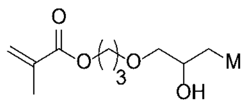 , 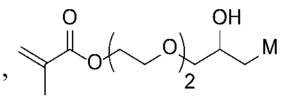 , 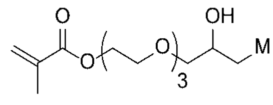 ,

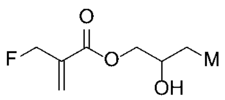 , 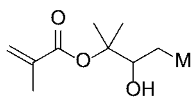 , 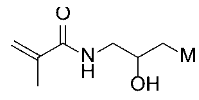 , 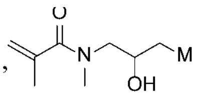 ,

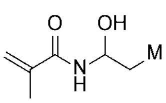 , 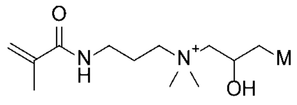 , 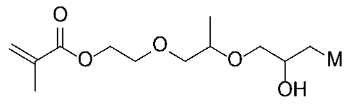 ,

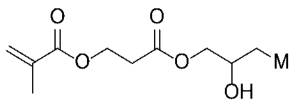 , 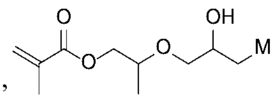 , 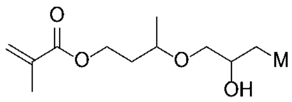 ,

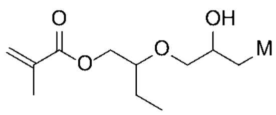 , 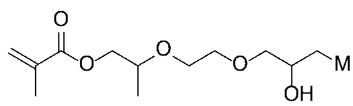

or

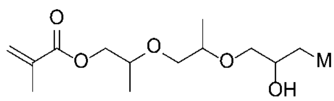 ,

wherein M is as defined above.

In some embodiments, the structural unit represented by formula II -b or formula II -B is derived from a monomeric compound selected from:

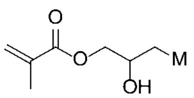 , 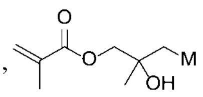 , 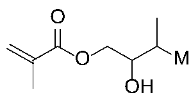 , 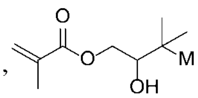 ,

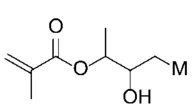 , 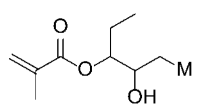 , 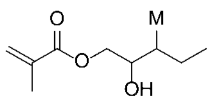 , 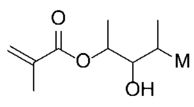 ,

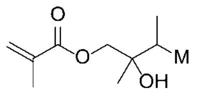 , 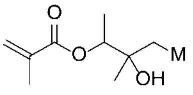 , 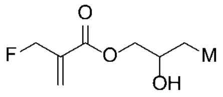 , 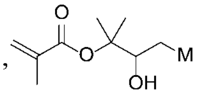 ,

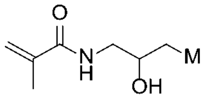 , 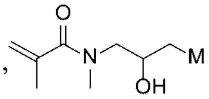 or 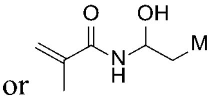 ,

wherein M is as defined above.

In some embodiments, the structural unit represented by formula III -b or formula III-B is derived from a monomeric compound selected from:

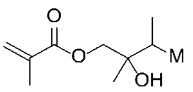 , 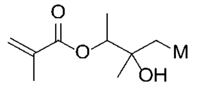 , 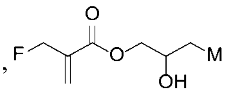 or 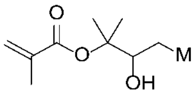 ,

wherein M is as defined above.

In some embodiments, the structural unit represented by formula I-c, formula I-C or formula I-C' is derived from a monomeric compound selected from:

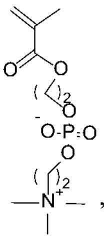 , 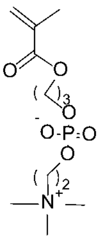 , 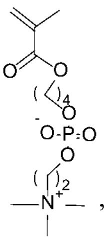 , 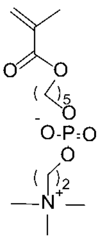 , 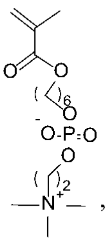 , 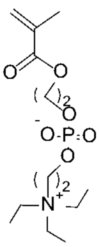 , 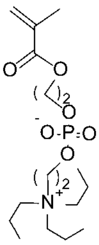 , 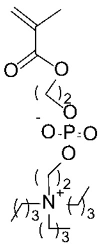 ,

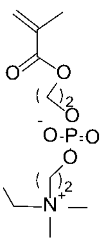 , 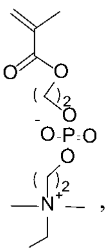 , 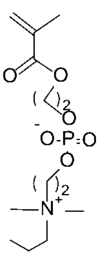 , 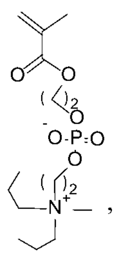 , 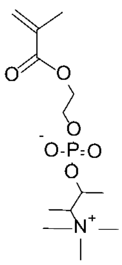 , 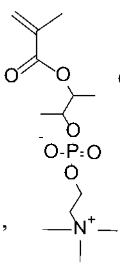 , 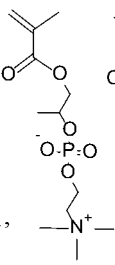 , 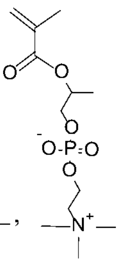 ,

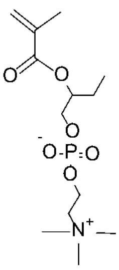 , 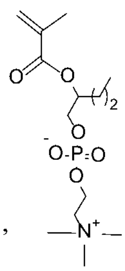 , 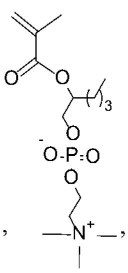 , 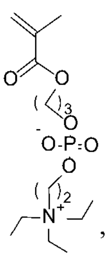 , 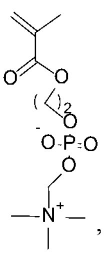 , 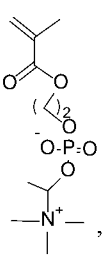 , 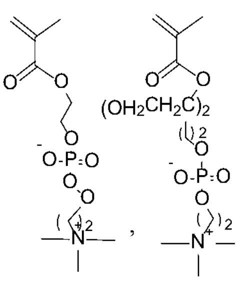 ,

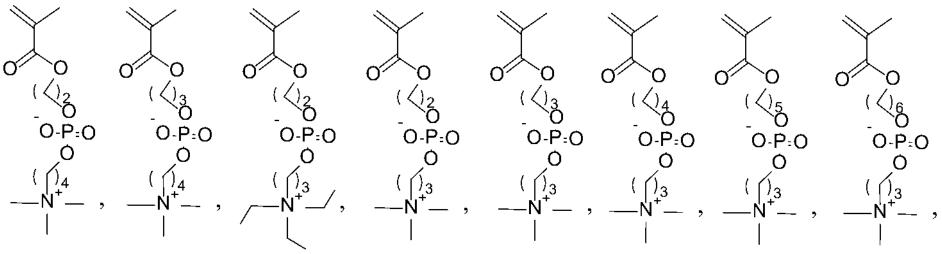

,

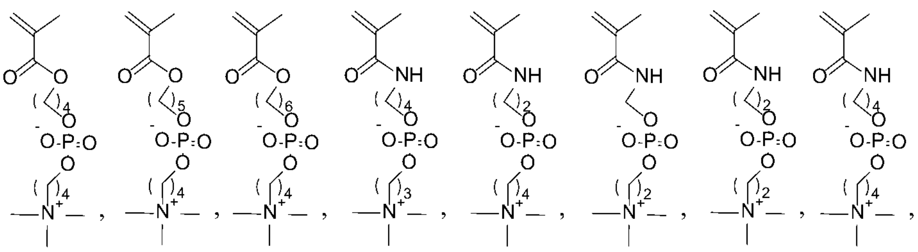

,

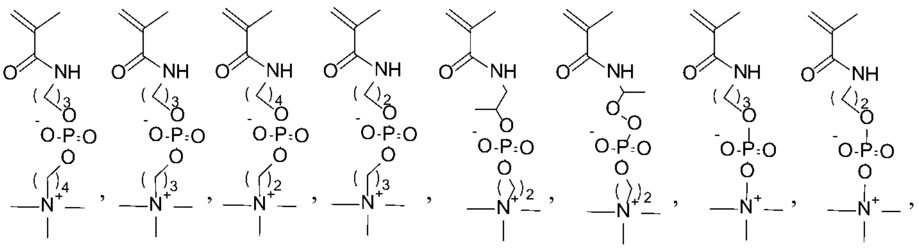

,

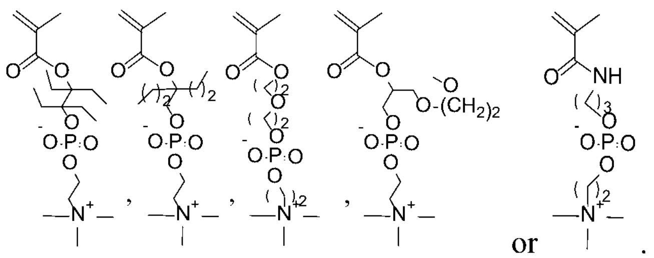

or .

In some embodiments, the structural unit represented by formula II -c, formula II -C or formula II -C' is derived from a monomeric compound selected from:

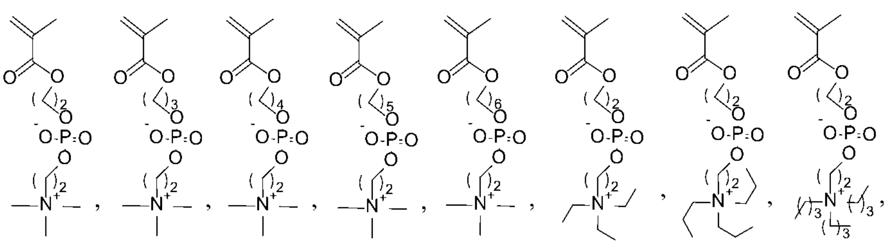

,

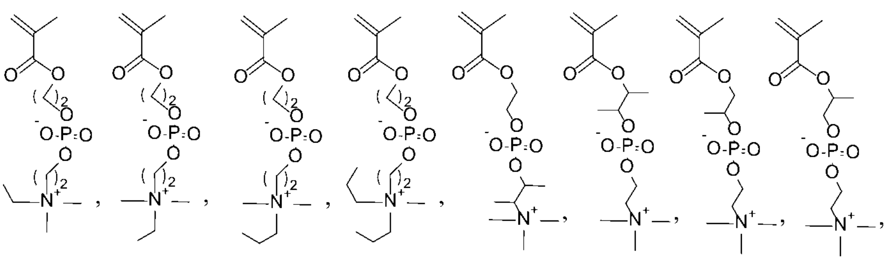

,

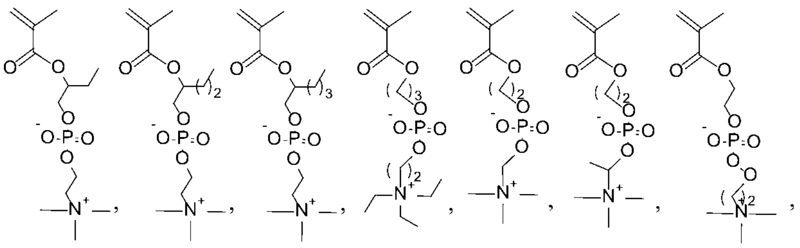

, , , , , ,

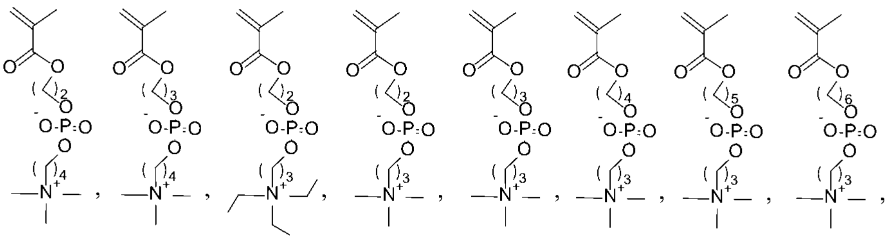

, , , , , , ,

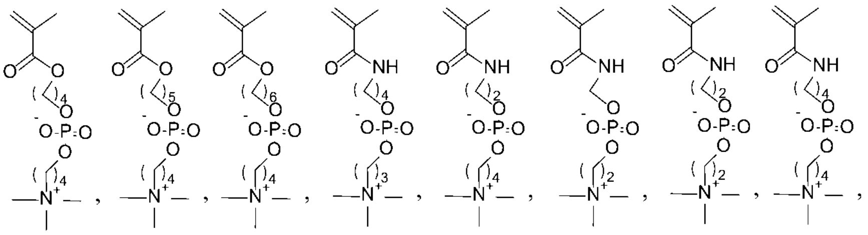

, , , , , , , ,

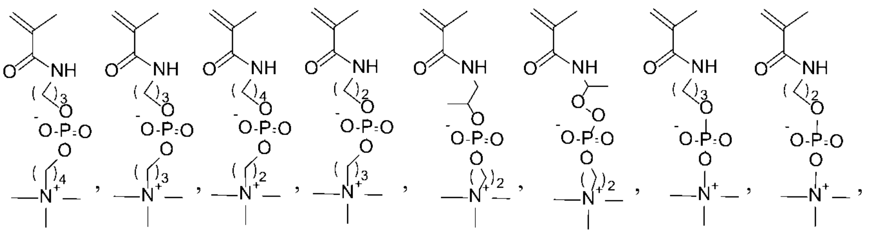

, , , , , , , ,

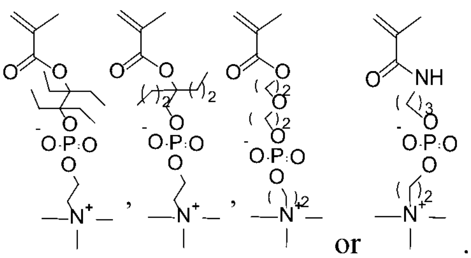

, , or .

In some embodiments, the structural unit represented by formula III-c, formula III-C or formula III-C' is derived from a monomeric compound selected from:

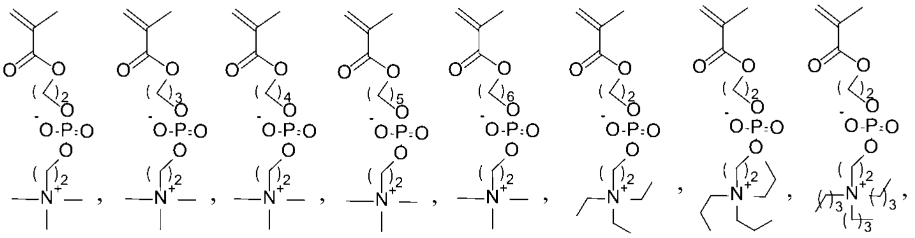

, , , , , , , ,

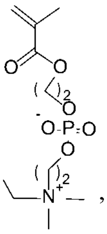, 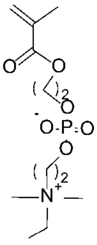, 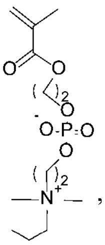, 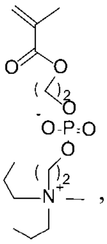, 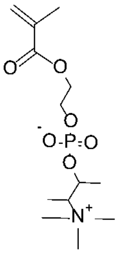, 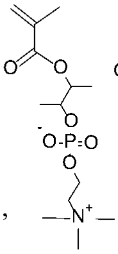, 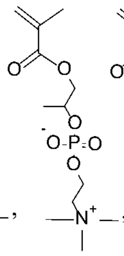, 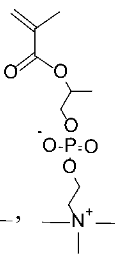,

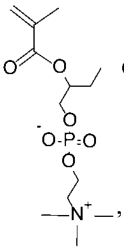, 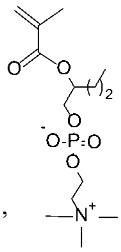, 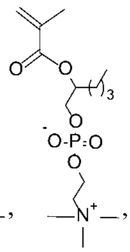, 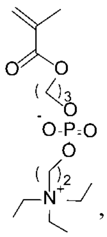, 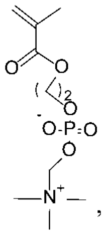, 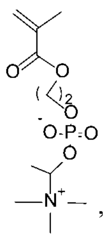, 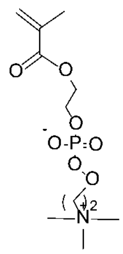,

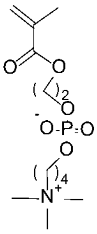, 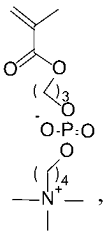, 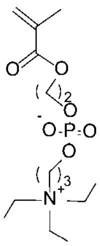, 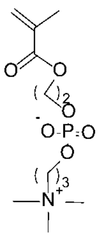, 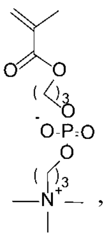, 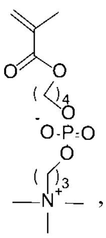, 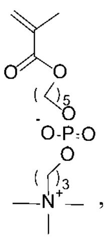, 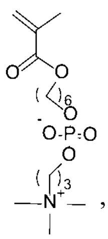,

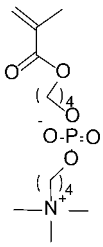, 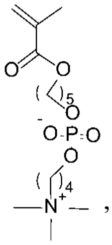, 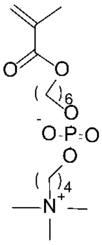, 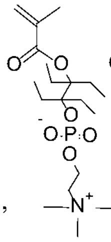, 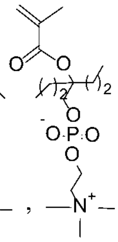 or 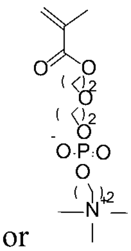.

In another aspect, the present disclosure further provides a method for preparing a biocompatible implantable material, comprising:

step 1) hydroxylating the surface of a substrate of a material;
step 2) treating the hydroxylated surface of the substrate with a coupling agent, which is cured to form an inner coating layer, i.e., a silane layer;
step 3) preparing the polymer defined above into a polymer solution, immersing the material treated in step 2) in the polymer solution, and then immersing the material in a solution of the amino acid (A) defined above, which is cured to form an outer coating layer, i.e., a polymer layer;
step 4) separating and removing any residual polymer from the surface of the substrate;
step 5) drying the material.

In some embodiments, in order to apply the polymer layer and/or optionally the silane layer to the substrate, the surface of the substrate of the material may first be washed. For example, the surface of the substrate may first be ultrasonicated and washed with a suitable solvent such as acetone, isopropanol, ethanol, methanol, or combinations thereof. Ultrasonication may occur for a period of time from about 1 minute to about 20 minutes, in some embodiments from about 5 minutes to about 15 minutes. However, in embodiments, ultrasonication may occur for longer periods of time, up to 1 hour, up to 2 hours, or for 2 hours or more. The solvents used for ultrasonication/washing may be applied as a mixture, or individual solvents may be applied sequentially one or more times. Ultrasonication may occur at room temperature (e.g., about 21°C) or at a temperature from about 18°C to about 55°C, in some embodiments from about 40°C to about 50°C, and

in some embodiments, about 45°C.

In some embodiments, the hydroxylation in step 1) occurs after the surface of the substrate is washed. The term "hydroxylation" or "substrate surface hydroxylation" refers to increasing the number of hydroxyl groups on the surface of the substrate. The process of "hydroxylation" or "substrate surface hydroxylation" refers to a method whereby the surface of the substrate to be treated can be hydroxylated by using a variety of oxidizing agents. The oxidizing agents include acids, bases, peroxides, plasma treatment agents, and combinations thereof. The acids include hydrochloric acid, nitric acid, sulphuric acid, hydrofluoric acid, perchloric acid, or combinations thereof. The bases include sodium hydroxide, ammonium hydroxide, potassium hydroxide, or combinations thereof. In certain embodiments, the oxidizing agent is a combination of an acid and a peroxide, preferably a combination of nitric acid and hydrogen peroxide, hydrochloric acid and hydrogen peroxide, or sulphuric acid and hydrogen peroxide, most preferably a combination of nitric acid and hydrogen peroxide. In certain embodiments, the oxidizing agent is a combination of a base and a peroxide, preferably a combination of sodium hydroxide and hydrogen peroxide, ammonium hydroxide and hydrogen peroxide, or potassium hydroxide and hydrogen peroxide, most preferably a combination of sodium hydroxide and hydrogen peroxide.

In certain embodiments, the concentration of the oxidizing agent applied may be from about 1% to about 100%. In certain embodiments, the concentration of the oxidizing agent applied is from about 15% to about 25%. In certain embodiments, the concentration of the oxidizing agent applied is about 20%.

In certain embodiments, the process of "hydroxylation" or "substrate surface hydroxylation" refers to a method whereby the surface of the substrate can be hydroxylated through treatment with sodium hydroxide, nitric acid, sulphuric acid, hydrochloric acid, ammonium hydroxide, hydrogen peroxide, tert-butyl hydroperoxide, potassium dichromate, perchloric acid, oxygen plasma, water plasma, corona discharge, ozone, UV, combinations thereof, and the like.

In certain embodiments, the hydroxylation reaction may occur at room temperature for a duration ranging from about 0.25 hours to about 4 hours. In certain embodiments, the hydroxylation reaction may occur at room temperature for a duration ranging from about 1 hour to about 2 hours. In certain embodiments, the hydroxylation reaction may occur at room temperature for a duration in embodiments ranging from about 1.5 hours.

In certain embodiments, the hydroxylation reaction may also occur under shaking at a speed of from about 100 rpm to about 160 rpm. In certain embodiments, the hydroxylation reaction may also occur under shaking at a speed of from about 120 rpm to about 140 rpm. In certain embodiments, the hydroxylation reaction may also occur under shaking at about 130 rpm.

In certain embodiments, the hydroxylation covers about 10%, about 20%, about 30%, about 40%, about 50%, about 60%, about 70%, about 80%, about 90%, or about 100% of the surface of the substrate.

After hydroxylation, the substrate may be washed with a suitable material such as deionized water, ethanol, methanol, or combinations thereof.

In some embodiments, the treatment method in step 2) comprises: soaking the washed surface of the substrate of the material in a coupling agent solution, followed by washing and subsequent drying. A silane layer is formed on the treated surface of the substrate.

In certain embodiments, suitable solvents in the solution used for forming the silane layer include, for example, ethanol, toluene, water, deionized water, methanol, isopropanol, n-butanol, dimethylformamide (DMF), dimethyl sulfoxide (DMSO), ethyl acetate, propylene glycol methyl ether acetate (PM acetate), trichloromethane, dichloromethane, combinations thereof, and the like.

In certain embodiments, the content of the solvent in the solution used for forming the silane layer may be from about 0.1 wt% to about 99.9 wt% of the solution. In certain embodiments, the content of the solvent may be from about 50 wt% to about 99.8 wt% of the solution. In certain embodiments, the solvent is ethanol/water (95%/5%).

In certain embodiments, the concentration of the coupling agent in the solution used for forming the silane layer may be from about 0.1 wt% to about 99.9 wt% of the solution. In certain embodiments, the concentration of the coupling agent is from about 0.2 wt% to about 50 wt% of the solution.

In certain embodiments, the substrate may be immersed in a solution comprising a coupling agent at room temperature for from about 0.5 hours to about 3.5 hours. In certain embodiments, the substrate may be immersed in a solution comprising a coupling agent at room temperature for from 1 hour to about 3 hours. In certain embodiments, the substrate may be immersed in a solution comprising a coupling agent at room temperature for about 2 hours.

In certain embodiments, the coupling agent solution used for treating the substrate may also be subjected to shaking at a speed of from about 100 rpm to about 160 rpm. In certain embodiments, the coupling agent solution used for treating the substrate may also be subjected to shaking at a speed of from about 120 rpm to about 140 rpm. In certain embodiments, the coupling agent solution used for treating the substrate may also be subjected to shaking at a speed of about 130 rpm.

In certain embodiments, after immersing in the coupling agent solution, the substrate may be soaked or sprayed with a suitable material (such as ethanol, toluene, water, deionized water, methanol, isopropanol, n-butanol, dimethylformamide (DMF), dimethyl sulfoxide (DMSO), ethyl acetate, PM acetate, trichloromethane, dichloromethane, or combinations thereof) from 1 to about 5 times, preferably about 3 times.

In certain embodiments, the surface of the substrate treated with the coupling agent is cured by heating and drying, and the heating temperature may be from about 30°C to about 150°C. In certain embodiments, the heating temperature is from about 60°C to about 100°C. In certain embodiments, the heating temperature is about 80°C. In certain embodiments, the heating may occur for from about 5 minutes to about 60 minutes, or for 60 minutes or more. In certain embodiments, the heating may occur for from about 15 minutes to about 45 minutes. In certain embodiments, the heating may occur for about 30 minutes.

In certain embodiments, the silane layer formed on the surface of the substrate may have a thickness of less than 50 nanometres, less than 20 nanometres, or less than 10 nanometres. In certain embodiments, the silane layer formed on the surface of the substrate has a thickness of from about 1 nanometre to about 10 nanometres.

In some embodiments, after the silane layer is formed, the material may be treated with additional components to form an outer coating layer on the silane layer. For example, a bioactive agent may bind to the reactive functional groups of the silane layer. Similarly, polymeric and/or monomeric materials may bind to the reactive functional groups on the substrate and/or the silane layer, either in the presence or absence of a bioactive agent. The term "reactive functional group" refers to a group that is highly reactive with a heterocyclyl group and can undergo a ring-opening reaction with the heterocyclyl group. The reactive functional groups are, for example, hydroxyl, carboxyl, mercapto or amino groups, or groups capable of being converted to contain hydroxyl, mercapto, amino or carboxyl groups. In certain embodiments, the heterocyclyl is an epoxy group, such as oxiranyl.

In some embodiments, suitable polymeric and/or monomeric materials that may be used for forming an outer coating layer on the material of the present disclosure, and that bind to the substrate, the aforementioned silane layer, or both, include any materials compatible with the material. These materials can impart desired properties to the material, including reduced procoagulant activity, lubricity, drug delivery, protein or DNA transfer, restenosis prevention, cell and protein adhesion, RNA and/or gene transfer, antimicrobial properties, antifouling properties, promotion of endothelialisation, combinations thereof, and the like.

In some embodiments, suitable polymeric and/or monomeric materials that may bind to the substrate and/or the aforementioned silane layer, and that used for forming an outer coating layer on the material of the present disclosure, include phosphorylcholine and heterocyclyl.

In some embodiments, a suitable polymer is capable of binding to the substrate and/or the aforementioned silane layer, and is used for forming an outer coating layer on the material of the present disclosure. The polymer is as defined above.

In some embodiments, the treatment method in step 3) comprises: immersing the substrate of the material treated with the coupling agent in a polymer solution, whereby the reactive functional groups on the coupling agent and the heterocyclyl groups on the polymer are covalently connected via a ring-opening reaction; then immersing the reacted substrate in an amino acid solution for treatment, ensuring complete ring-opening of all unreacted heterocyclyl groups in the polymer after sufficient reaction; and after curing treatment, forming a polymer layer as the outermost coating layer on the surface of the substrate.

In some embodiments, the polymer used for forming the polymer layer may be in a solution, which is then applied to the substrate and/or the aforementioned silane layer. Suitable solvents used for forming the solution for treating the polymer include, for example, ethanol, water, deionized water, methanol, isopropanol, n-butanol, dimethylformamide (DMF), dimethyl sulfoxide (DMSO), ethyl acetate, PM acetate, toluene, trichloromethane, dichloromethane, combinations thereof, and the like.

In certain embodiments, the concentration of the polymer solution used for forming the polymer layer may be from about 0.5 wt% to about 95 wt% of the solution. In certain embodiments, the concentration of the polymer solution is from about 1 wt% to about 50 wt% of the solution.

The polymer may be applied to the substrate and/or optionally the silane layer using a variety of methods including soaking, spraying, brushing, combinations thereof, and the like. For example, in certain embodiments, the substrate with the silane layer may be immersed in a solution containing a polymer at room temperature for a duration of from about 30 seconds to about 60 minutes. In certain embodiments, the duration is from about 1 minute to about 30 minutes. In certain embodiments, the duration is about 45 seconds, about 60 seconds, about 75 seconds, about 90 seconds, about 2 minutes, about 3 minutes, about 4 minutes, about 5 minutes, about 6 minutes, about 7 minutes, about 8 minutes, about 9 minutes, or about 10 minutes.

In certain embodiments, the concentration of the amino acid solution may be from about 0.1 wt% to about 99.9 wt% of the solution. In certain embodiments, the concentration of the amino acid solution is from about 1 wt% to about 50 wt% of the solution.

The substrate that has been soaked in the polymer solution may be immersed in the solution comprising the polymer again at room temperature for a duration of from about 1 hour to about 48 hours. In certain embodiments, the duration is about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23

hours, or about 24 hours.

In certain embodiments, the surface of the substrate treated with the amino acid solution is cured by heating and drying, and the heating temperature may be from about 30°C to about 150°C. In certain embodiments, the heating temperature is from about 60°C to about 100°C. In certain embodiments, the heating temperature is about 80°C. In certain embodiments, the heating may occur for from about 5 minutes to about 60 minutes, or for 60 minutes or more. In certain embodiments, the heating may occur for from about 15 minutes to about 45 minutes. In certain embodiments, the heating may occur for about 30 minutes.

The cured material may be washed again. For example, the material may be ultrasonicated and washed with water, ethanol, methanol, isopropanol, n-butanol, dimethylformamide (DMF), dimethyl sulfoxide (DMSO), ethyl acetate, PM acetate, toluene, trichloromethane, dichloromethane, combinations thereof, and the like. Ultrasonication may occur for a duration of from about 1 minute to about 10 minutes, or for 10 minutes or more. In certain embodiments, the duration is from about 2 minutes to about 8 minutes. In certain embodiments, the duration is about 5 minutes. Ultrasonication may occur at room temperature.

After this washing, the material may be rinsed with a suitable material (such as water, methanol, isopropanol, n-butanol, dimethylformamide (DMF), dimethyl sulfoxide (DMSO), ethyl acetate, PM acetate, toluene, trichloromethane, dichloromethane, or combinations thereof). The material is then heated and dried, and the heating temperature may be from about 30°C to about 150°C. In certain embodiments, the heating temperature is from about 60°C to about 100°C. In certain embodiments, the heating temperature is about 80°C. In certain embodiments, the heating may occur for from about 5 minutes to about 60 minutes, or for 60 minutes or more. In certain embodiments, the heating may occur for from about 15 minutes to about 45 minutes. In certain embodiments, the heating may occur for about 30 minutes.

The polymer layer on the resulting material may have a thickness of less than about 2000 nanometres. In certain embodiments, it has a thickness of less than about 1000 nanometres. In certain embodiments, it has a thickness of less than about 500 nanometres. In certain embodiments, it has a thickness of less than about 250 nanometres. In certain embodiments, it has a thickness of less than about 100 nanometres. In certain embodiments, it has a thickness of less than about 50 nanometres. In certain embodiments, it has a thickness of less than about 25 nanometres. In certain embodiments, it has a thickness of less than about 10 nanometres. In certain embodiments, it has a thickness of less than about 1 nanometre to about 100 nanometres. In certain embodiments, it has a thickness of less than about 1 nanometre to about 50 nanometres. In certain embodiments, it has a thickness of less than about 1 nanometre to about 25 nanometres. In certain embodiments, it has a thickness of less than about 1 nanometre to about 10 nanometres. In certain embodiments, the polymer layer is the outermost coating layer of the material and/or any components forming the material (e.g., filaments forming a stent), and/or forms the outer coating layer thereof.

As mentioned above, a bioactive agent may be added to the materials of the present disclosure as part of the material and/or as part of a layer applied according to the present disclosure. As used herein, "bioactive agent" includes any substance or mixture of substances that provides a therapeutic or prophylactic effect; a compound that affects or participates in tissue growth, cell growth and/or cell differentiation; a compound that may be able to cause or prevent a biological effect (such as an immune response); or a compound that may play any other role in one or more biological processes. A variety of bioactive agents may be incorporated into the material. Furthermore, any agent that can enhance tissue repair, limit the risk of restenosis, and modulate the mechanical or physical properties of a material such as a stent may be added during preparation of the material. In certain embodiments, a bioactive agent may be added to the polymer used for forming the outer coating layer of the material.

Examples of categories of bioactive agents that may be utilized according to the present disclosure include antimicrobial agents, analgesics, anaesthetics, antihistamines, anti-inflammatory drugs, cardiovascular drugs, diagnostic agents, sympathomimetics, cholinergics, antimuscarinics, antispasmodics, hormone preparations, growth factors, muscle relaxants, adrenergic blockers, antineoplastic agents, immunogenic agents, immunosuppressants, steroids, lipids, lipopolysaccharides, polysaccharides, and enzymes. It is also contemplated that combinations of bioactive agents may be used.

The materials described in the present disclosure include, but are not limited to, stents, filters, stent coatings, grafts, catheters, stent-graft analogues, clamps and other fasteners, staples, sutures, pins, screws, prosthetic materials, drug delivery materials, anastomotic rings, surgical blades, contact lenses, intraocular lenses, surgical meshes, knotless wound closure materials, sealants, adhesives, anti-adhesion materials, anchors, conduits, bone fillers, synthetic tendons, synthetic ligaments, tissue scaffolds, anastomotic materials, hinge plates, abdominal bands, orthopaedic hardware, pacers/pacemakers, and other implants and implantable materials.

In some embodiments, the material is a stent, a stent-graft, or a stent analogue. Any stent may be processed according to the methods described herein. The stent may be a braided stent or other types of stents, such as laser-cut stents, roll-formed stents, balloon-expandable stents, self-expanding stents, or knitted stents.

In certain embodiments, a braided vascular material, such as a stent, is formed by braiding filaments made from metal alloys and/or other high-temperature materials. The resulting braided structure is then subjected to heat treatment or "heat-setting" at elevated temperatures to reduce internal stress within the filaments and/or to enhance or impart self-expanding properties to the stent. The filaments constituting the tubular body of a heat-set stent are in a state of minimal or reduced stress when the stent is maintained in the configuration adopted during the heat-setting process. This state of minimal or reduced stress may include an expanded or fully expanded state. The stent may optionally be configured to function as a "flow diverter" for the treatment of aneurysms, such as those found in blood vessels including cerebral or intracranial arteries, or in other locations within the human body (such as peripheral arteries).

For example, according to the present disclosure, a material comprising a flow-diverting segment may possess pores with a "flow-diverting pore size." The "flow-diverting pore size" may refer to an average pore size (in at least one segment of the material) that is sufficiently small to disrupt or prevent fluid exchange through the pores of that segment. For example, a material (e.g., a stent) may comprise a functional segment or flow-diverting segment with a flow-diverting pore size when the pores in the flow-diverting segment are sized such that-with the material/stent positioned within a blood vessel and adjacent to or across the neck of an aneurysm-blood flow through the sidewall into the aneurysm is prevented to an extent sufficient to induce thrombosis or heal the aneurysm.

For instance, a flow-diverting pore size may be achieved when the pores in the flow-diverting or functional segment (or in the stent as a whole), while the material (e.g., the stent) is in an expanded state, exhibit an average pore size smaller than about 500 micrometres. (When the term "expanded state" is used herein to specify parameters of a braided stent, such as pore size, it refers to the state in which the stent has self-expanded without the application of any external expansion force, and without the application of any external longitudinal tensile or compressive force. For the sake of measurement simplicity, this expanded state may be defined as the state in which the stent is allowed to self-expand within an upright cylindrical glass tube having an inner diameter smaller than the maximum diameter (i.e., the diameter to which the stent would self-expand in the absence of any constraining or external forces)). In some embodiments, the average pore size may be less than about 320 micrometres. Some embodiments disclosed herein achieve and provide a material and a manufacturing method, wherein the material comprises a flow-diverting segment or flow-diverting sidewall with reduced thrombogenicity, or wherein the material possesses overall flow-diverting properties and reduced thrombogenicity.

Therefore, in some embodiments, a material (such as a braided stent) is provided that may comprise a flow-diverting segment or another portion which imparts embolic properties so as to disrupt blood flow within or across a body space (e.g., an aneurysm) where the material is deployed, or into the body space (e.g., the aneurysm). The porosity and/or pore size of one or more segments of the material may be selected to disrupt blood flow to an extent sufficient to induce thrombosis in an aneurysm or other body space.

For example, some embodiments provide a material (e.g., a stent) that may be configured to disrupt blood flow so as to substantially reduce blood exchange between the parent vessel and the aneurysm, which may lead to thrombosis within the aneurysm. Therefore, a material (or a material component, such as a stent sidewall or a segment of such a sidewall) that disrupts blood flow may be regarded as possessing "flow-diverting" properties.

Furthermore, in some embodiments, the material (e.g., the stent) may have a porosity ranging from 5% to 95% and may be employed in an expanded braided structure. In some embodiments, a porosity ranging from 30% to 90% may be employed. Furthermore, a porosity ranging from 50% to 85% may be employed. Porosity may be calculated as the percentage of the total external surface area in a stent that is open, where the total external surface area is the sum of the open surface area (occupied by pores) and the solid surface area (occupied by filaments).

Furthermore, in some embodiments, the material (e.g., the stent) may have a pore size of from about 20 micrometres to 300 micrometres (inscribed diameter). In some embodiments, a pore size of from about 25 micrometres to 250 micrometres (inscribed diameter) may be employed. In some embodiments, a pore size of from about 50 micrometres to 200 micrometres (inscribed diameter) may be employed.

In some embodiments, the surface of the substrate of the material used for the coating may be composed of optional surface materials including metals, glass, polymers, ceramics, combinations thereof, and the like. In certain embodiments, the surface material is metal. Although any metal surface may be used, suitable metals may include gold, silver, copper, steel, aluminium, titanium, cobalt, chromium, platinum, nickel, alloys thereof, and combinations thereof. Suitable alloys may include Nitinol (nickel titanium), cobalt nickel, cobalt chromium, platinum tungsten, and combinations thereof. In certain embodiments, the surface material may be activated by hydroxylation.

In some embodiments, at least a portion of the surface of the substrate of the material is coated with the coating as described herein. The coating comprises a polymer layer and/or a silane layer. The coating may cover from about 1% to about 100% of the surface of the substrate. In certain embodiments, at least about 10%, at least about 20%, at least about 30%, at least about 40%, at least about 50%, at least about 60%, at least about 70%, at least about 80%, at least about 90%, at least about 95%, at least about 95%, or about 100% of the surface of the substrate may be coated.

The present disclosure also encompasses various deuterated forms of the polymer or pharmaceutically acceptable salt thereof, wherein each available hydrogen atom in the polymer may be independently replaced by a deuterium atom. Those skilled in the art will know how to synthesise deuterated forms of the polymer of the present disclosure or pharmaceutically acceptable salt thereof.

The present disclosure also includes isotopically labelled polymers, wherein one or more atoms in the polymer

are replaced by an atom having an atomic mass or mass number different from the atomic mass or mass number typically found in nature. Examples of isotopes useful in the polymers of the present disclosure include isotopes of hydrogen, carbon, nitrogen, oxygen, fluorine, iodine, and chlorine, such as $^{3}H$, $^{11}C$, $^{14}C$, $^{18}F$, $^{123}I$ or $^{125}I$.

The polymers of the present disclosure may be in the form of pharmaceutically acceptable salts. However, it is to be understood that non-pharmaceutically acceptable salts also fall within the scope of the present disclosure, as these may be useful as intermediates in the preparation of pharmaceutically acceptable salts or may be useful during storage or transport. Suitable pharmaceutically acceptable salts include, but are not limited to, salts of pharmaceutically acceptable inorganic acids (such as hydrochloric acid, sulphuric acid, phosphoric acid, nitric acid, carbonic acid, boric acid, sulfamic acid, and hydrobromic acid), or salts of pharmaceutically acceptable organic acids (such as acetic acid, propionic acid, butyric acid, tartaric acid, maleic acid, hydroxymaleic acid, fumaric acid, citric acid, lactic acid, mucic acid, gluconic acid, benzoic acid, succinic acid, oxalic acid, phenylacetic acid, methanesulfonic acid, toluenesulphonic acid, benzenesulfonic acid, salicylic acid, sulphanilic acid, aspartic acid, glutamic acid, ethylenediaminetetraacetic acid, stearic acid, palmitic acid, oleic acid, lauric acid, pantothenic acid, tannic acid, ascorbic acid, and valeric acid). Basic salts include, but are not limited to, those formed with pharmaceutically acceptable cations such as sodium, potassium, lithium, calcium, magnesium, ammonium, and alkylammonium.

Unless stated to the contrary, the following terms used in the description and claims have the following meanings.

The term "halogen" refers to fluorine, chlorine, bromine or iodine.

The term "hydroxyl" refers to -OH.

The term "amino" refers to $-NH_2$.

The term "cyano" refers to -CN.

The term "nitro" refers to $-NO_2$.

The term "oxo group" or "oxo" refers to "=O".

The term "thio group" or "thio" refers to "=S".

The term "carbonyl" refers to C=O.

The term "carboxyl" refers to -C(O)OH.

The term "halo" refers to substitution with one or more atoms selected from fluorine, chlorine, bromine, and iodine.

The term "alkyl" refers to a linear or branched saturated hydrocarbon group having 1 to 6 carbon atoms. Where appropriate, the alkyl may have a specified number of carbon atoms, for example, $C_{1-4}$ alkyl, which includes alkyl groups having 1, 2, 3 or 4 carbon atoms in a linear or branched arrangement. Suitable examples of alkyl include, but are not limited to, methyl, ethyl, n-propyl, isopropyl, n-butyl, isobutyl, tert-butyl, n-pentyl, 2-methylbutyl, 3-methylbutyl, 4-methylbutyl, n-hexyl, 2-methylpentyl, 3-methylpentyl, 4-methylpentyl, 5-methylpentyl, 2-ethylbutyl, 3-ethylbutyl, heptyl, octyl, nonyl, and decyl. Unless otherwise specified, the alkyl may be substituted or unsubstituted.

The term "hydroxyalkyl" refers to alkyl substituted with one or more hydroxyl groups, wherein the alkyl is as defined above.

The term "aminoalkyl" refers to alkyl substituted with one or more amino groups, wherein the alkyl is as defined above.

The term "carboxyalkyl" refers to alkyl substituted with one or more carboxyl groups, wherein the alkyl is as defined above.

The term "haloalkyl" refers to alkyl substituted with one or more halogen (such as fluorine, chlorine, bromine, and iodine), wherein the alkyl is as defined above.

The term "alkoxy" refers to -O-(alkyl), wherein the alkyl is as defined above. Non-limiting examples of alkoxy include: methoxy, ethoxy, propoxy, butoxy, cyclopropoxy, cyclobutoxy, cyclopentoxy, and cyclohexoxy. Unless otherwise specified, the alkoxy may be substituted or unsubstituted.

The term "haloalkoxy" refers to alkoxy substituted with one or more halogen (such as fluorine, chlorine, bromine, and iodine), wherein the alkoxy is as defined above.

The term "aminoalkoxy" refers to alkoxy substituted with one or more amino groups, wherein the alkoxy is as defined above.

The term "hydroxyalkoxy" refers to alkoxy substituted with one or more hydroxy groups, wherein the alkoxy is as defined above.

The term "monovalent group" refers to a group formed by "formally" eliminating a monovalent atom or group from a compound.

The term "-ylene" refers to a group formed by "formally" eliminating two monovalent atoms or atomic groups or one divalent atom or atomic group from a compound.

The term "alkylene" refers to the moiety remaining after removal of two hydrogen atoms from an alkane molecule, including linear and branched -ylene groups comprising 1 to 20 carbon atoms. Non-limiting examples include methylene ($-CH_2-$) and ethylene (such as $-CH_2CH_2-$ or $-CH(CH_3)-$). Unless otherwise specified, the alkylene may be substituted or unsubstituted.

The term "heteroalkylene" refers to alkylene in which one or more -$CH_2$-groups are replaced by a heteroatom selected from N, O, and S, wherein the alkylene is as defined above. Unless otherwise specified, the heteroalkylene may be substituted or unsubstituted.

The term "cycloalkyl" or "carbocyclic ring" refers to a saturated or unsaturated cyclic hydrocarbon. The cycloalkyl ring may include the specified number of carbon atoms. For example, 3- to 8-membered cycloalkyl includes 3, 4, 5, 6, 7, or 8 carbon atoms. Suitable examples of cycloalkyl include, but are not limited to, cyclopropyl, cyclobutyl, cyclopentyl, cyclopentenyl, cyclohexyl, cyclohexenyl, 1,4-cyclohexadienyl, cycloheptyl and cyclooctyl. Unless otherwise specified, the cycloalkyl or carbocyclic ring may be substituted or unsubstituted.

The term "cycloalkylene" refers to a divalent cyclic hydrocarbon group derived from a cycloalkyl group, e.g.,

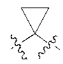 , 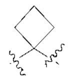 , 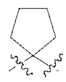 , 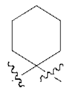 ,

etc. Unless otherwise specified, the cycloalkylene group may be substituted or unsubstituted.

The term "heterocycloalkyl," "heterocyclyl," or "heterocyclic ring" refers to a cyclic hydrocarbon in which one to four carbon atoms have been replaced by heteroatoms independently selected from N, N(R), S, S(O), $S(O)_2$, and O. The heterocyclic ring may be saturated or unsaturated. Suitable examples of heterocyclyl include oxiranyl, tetrahydrofuryl, tetrahydrothienyl, pyrrolidyl, pyrrolinyl, pyrazolinyl, pyranyl, piperidyl, dithiolyl, oxathiolyl, dioxanyl, dioxinyl, morpholinyl and oxazinyl. Unless otherwise specified, the heterocycloalkyl or heterocyclic ring may be substituted or unsubstituted.

The term "heterocyclylene" refers to a divalent heterocyclyl, wherein the heterocyclyl is as defined above. The heterocyclylene includes, but is not limited to, piperidine-1,4-diyl, piperazine-1,4-diyl, tetrahydrofuran-2,4-diyl, tetrahydrofuran-3,4-diyl, azetidine-1,3-diyl, and pyrrolidine-1,3-diyl, wherein the heterocyclyl is as defined above.

The term "aryl" or "aromatic ring" refers to a mono- or bicyclic aromatic ring system containing 6 to 12 ring carbon atoms. Examples of aryl include, but are not limited to, phenyl and naphthyl. The term "aryl" as described in the present invention may include fused ring systems, including, for example, ring systems in which an aromatic ring is fused to a cycloalkyl ring. Examples of such fused ring systems include, for example, 1,2-dihydroindene and indene. Unless otherwise specified, the aryl or aromatic ring may be substituted or unsubstituted.

The term "arylene" refers to a group derived by the removal of two hydrogen atoms from the same or two different carbon atoms of a parent aryl group. The arylene includes, but is not limited to, phenylene, wherein the aryl is as defined above.

The term "heteroaryl" or "heteroaromatic ring" refers to aryl containing 5 to 10 ring carbon atoms in which one or more ring carbon atoms are replaced by at least one heteroatom such as -O-, -N-, or -S-, wherein the "aryl" is as defined above. Heteroaryl groups include, but are not limited to, acridinyl, carbazolyl, cinnolinyl, quinoxalinyl, quinazolinyl, pyrazolyl, indolyl, benzotriazolyl, furyl, thienyl, thiophenyl, 3,4-propylenedioxythiophenyl, benzothienyl, benzofuryl, benzodioxane, benzodioxine, quinolyl, isoquinolyl, oxazolyl, isoxazolyl, imidazolyl, pyrazinyl, pyridazinyl, pyridyl, pyrimidyl, pyrrolyl, tetrahydroquinoline, thiazolyl, isothiazolyl, 1,2,4-triazolyl, 1,2,3-triazolyl, 1,2,4-oxadiazolyl, 1,2,4-thiadiazolyl, 1,3,5-triazinyl, 1,2,4 triazinyl, 1,2,4,5 tetrazinyl and tetrazolyl. Unless otherwise specified, the heteroaryl or heteroaromatic ring may be substituted or unsubstituted.

The term "heteroarylene" refers to a group derived by the removal of two hydrogen atoms from the same or two different atoms of a parent heteroaryl group. The heteroarylene includes, but is not limited to, pyridylene, pyrrolylene, thiazolylene, imidazolylene, etc., wherein the heteroaryl is as defined above.

The term "optionally" or "optional" means that the event or circumstance subsequently described may but not necessarily occur, and the description includes the occasion where the event or circumstance occurs or does not occur. For example, "the surface of the substrate is optionally treated with a modifier" means that the surface of the substrate may be, but is not required to be, treated with a modifier. The term "all oxiranyl groups" refers to all the oxiranyl groups carried in the polymer. For example, "all oxiranyl groups of formula 1a are covalently connected to the reactive functional groups via a ring-opening reaction" means that all the oxiranyl groups contained in all the structural units of formula 1a in the polymer react fully with the reactive functional groups, so that all the oxiranyl groups are covalently bonded to the reactive functional groups via a ring-opening reaction.

The term "substituted" means that one or more hydrogen atoms, preferably at most 5, more preferably 1-3 hydrogen atoms in the group are each independently substituted with a corresponding number of substituents. It goes without saying, the substituents may be only in possible chemical positions thereof, and those skilled in the art can determine the possible or impossible substitutions (by experiment or theory) without paying too much effort.

The term "substituent" includes, but is not limited to, halogen, hydroxyl, oxo, amino, cyano, $C_{1-6}$ alkyl, $C_{1-6}$ alkoxy, halogenated $C_{1-6}$ alkyl, 3- to 6-membered cycloalkyl, 3- to 6-membered heterocycloalkyl, 6- to 10-membered aryl, or 5- to 10-membered heteroaryl.

The term "pharmaceutical composition" denotes a mixture containing one or more compounds described herein

or physiologically/pharmaceutically acceptable salts or prodrugs thereof and other chemical components, as well as other components, such as physiologically/ pharmaceutically acceptable carriers and excipients. The purpose of pharmaceutical compositions is to facilitate administration to living organisms and facilitate the absorption of active ingredients to exert biological activity.

With respect to drugs or pharmacologically active agents, the term "effective amount" refers to a non-toxic but sufficient amount of the drug or agent to achieve the desired effect. Determination of the effective amount, varying from person to person, depends on the age and general condition of a subject and also depends on a specific active substance. The appropriate effective amount in individual cases can be determined by those skilled in the art according to routine experiments.

The term "pharmaceutically acceptable" refers to those compounds, materials, compositions and/or dosage forms that are, within the scope of sound medical judgment, suitable for use in contact with patient tissues without excessive toxicity, irritation, allergic response, or other problems or complications, commensurate with a reasonable benefit/risk ratio, and effective for the intended use.

As used herein, the singular forms "a," "an," and "the" include plural referents and vice versa, unless otherwise clearly indicated in the context.

In the chemical structures of the compounds of the present disclosure, the bond "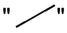" indicates that the configuration is not specified, that is, the bond "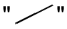" may be "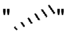" or "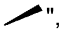", or contains both "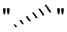" and "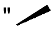" configurations. In the chemical structures of the compounds of the present disclosure, the bond "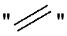" indicates that the configuration is not specified, that is, it may be a Z or E configuration, or contains both configurations.

Any isotopically labelled derivatives of the compounds of the present disclosure or pharmaceutically acceptable salts or isomers thereof are encompassed by the present disclosure. Atoms that may be isotopically labelled include, but are not limited to, hydrogen, carbon, nitrogen, oxygen, phosphorus, fluorine, chlorine, iodine, etc. They may be replaced by isotopes such as $^{2}H(D)$, $^{3}H$, $^{11}C$, $^{13}C$, $^{14}C$, $^{15}N$, $^{18}F$, $^{31}P$, $^{32}P$, $^{35}S$, $^{36}Cl$ and $^{125}I$, respectively. Unless otherwise specified, when a position is specifically designated as deuterium (D), it should be understood that the position has a deuterium abundance at least 3000 times greater than the natural abundance of deuterium (which is 0.015%) (i.e., at least 45% deuterium incorporation).

## Brief description of the drawings

Figure 1 shows the connection between the coupling agent, the polymer and the surface of the metal substrate.
Figure 2 is the $^{1}H$-NMR spectrum of polymer P(GMA-MPC).

## Detailed Description of Embodiments

The present disclosure is further described and explained below in conjunction with examples, but these examples are not intended to limit the scope of the present disclosure.

Experimental methods without specific conditions indicated in the examples of the present disclosure are generally conducted under conventional conditions, or under conditions recommended by the manufacturers of the starting materials or commercial products. The reagents for which the specific source is not indicated, are conventional commercially available reagents.

The structures of compounds are determined by nuclear magnetic resonance (NMR) or mass spectrometry (MS). NMR determination is carried out by Bruker AVANCE-400 nuclear magnetic resonance spectrometer; the solvents for determination are deuterated dimethyl sulfoxide (*DMSO-d6*), deuterated chloroform ($CDCl_3$) and deuterated methanol ($CD_3OD$); the internal standard is tetramethylsilane (TMS); and the chemical shift is given in $10^{-6}$ (ppm).

MS determination is carried out by FINNIGAN LCQAd (ESI) mass spectrometer (manufacturer: Thermo, model: Finnigan LCQ advantage MAX).

High performance liquid chromatography (HPLC) determination is carried out using Agilent 1200DAD high-pressure liquid chromatography instrument (Sunfire C18 150 × 4.6 mm chromatographic column) and Waters 2695-2996 high-pressure liquid chromatography instrument (Gimini C18 150 × 4.6 mm chromatographic column).

Yantai Huanghai HSGF254 or Qingdao GF254 silica gel plate is used as a thin layer chromatography silica plate, and the silica gel plate for thin layer chromatography (TLC) has a specification of 0.15 mm-0.2 mm, and the specification when separating and purifying a product by thin layer chromatography is 0.4 mm-0.5 mm.

Yantai Huanghai silica gel of 200-300 mesh is generally used as a carrier in column chromatography.

Known starting materials in the present disclosure may be synthesised using or according to methods known in the art, or may be purchased from ABCR GmbH & Co.KG, Acros Organnics, Aldrich Chemical Company, Accela ChemBio

Inc., Chembee Chemicals, and other companies.

Unless otherwise specified in the examples, all reactions are carried out under argon atmosphere or nitrogen atmosphere.

The argon atmosphere or nitrogen atmosphere means that the reaction bottle is connected to an argon or nitrogen balloon with a volume of about 1 L.

The hydrogen atmosphere means that the reaction bottle is connected to a hydrogen balloon with a volume of about 1 L.

In a pressurized hydrogenation reaction, Parr 3916EKX hydrogenator and Qinglan QL-500 hydrogen generator or HC2-SS hydrogenator are used.

Hydrogenation reactions are typically performed by evacuating the system and refilling it with hydrogen gas, repeating this operation three times.

In a microwave reaction, CEM Discover-S 908860 microwave reactor is used.

Unless otherwise specified in the examples, the solution in the reaction refers to an aqueous solution.

Unless otherwise specified in the examples, the reaction temperature is room temperature.

Room temperature is the most suitable reaction temperature, and the temperature range is from 20°C to 30°C.

Preparation of PBS buffer with pH = 6.5 in the examples: 8.5 g of $KH_2PO_4$, 8.56 g of $K_2HPO_4.3H_2O$, 5.85 g of NaCl, and 1.5 g of EDTA were placed in a bottle, the volume was adjusted to 2 L, and the mixture was dissolved completely by ultrasonication and shaken well, to afford the buffer.

Eluent systems for column chromatography and developing agent systems for thin layer chromatography, which are used to purify compounds, include: A: dichloromethane and isopropanol system, B: dichloromethane and methanol system, and C: petroleum ether and ethyl acetate system. The volume ratios of the solvents are adjusted according to the polarity of compounds, and can also be adjusted by adding a small amount of triethylamine and acidic or basic reagents.

Some compounds of the present disclosure are characterised by Q-TOF LC/MS. Q-TOF LC/MS is carried out using Agilent 6530 accurate-mass quadrupole-time-of-flight mass spectrometer and Agilent 1290-Infinity ultra-performance liquid chromatography instrument (Agilent Poroshell 300SB-C8 5 μm, 2.1 × 75 mm chromatographic column).

Example 1. Preparation of a P(GMA-MPC) solution

Step 1) synthesis of a polymer

3.0 g of MPC (purchased from TCI, lot no. KDW4G-PB) and 0.36 g of GMA (purchased from Adamas, lot no. P1909690) were dissolved in 50.0 ml of anhydrous ethanol, placed in a three-necked flask equipped with a thermometer and a condenser, and purged with nitrogen for 30 min. Thereafter, 0.1 g of azobisisobutyronitrile (purchased from Adamas, lot no. P1871519) was added at 60°C, and a polymerisation reaction was carried out for 10 to 20 hours. After the polymerisation, a polymer was precipitated in tetrahydrofuran and recovered as a white powder by vacuum drying at 40°C. $^1H$ NMR analysis confirmed that the obtained polymer contained 70 mol% MPC.

$^1H$-NMR (400MHz, $CD_3OD$): δ 4.35, $-COOCH_2CH-$; δ 4.24, $-COOCH_2CH_2O-$; δ 4.11, $-OCH_2CH_2N(CH_3)_3$; δ 3.76, $-OCH_2CH_2N(CH_3)_3$; δ 3.33, $-CH_2N(CH_3)_3$; δ 3.14, $-CHOCH_2$; δ 2.91 and δ 2.74, $-CHOCH_2$; δ 2.00 and δ 1.92, $-CH_2-C$.

Determination of the molecular weight of the polymer

2 mg of the obtained polymer was dissolved in 1 g of 0.1 mol/L sodium nitrate aqueous solution.

Test conditions: column: Shodex (SB-806M HQ), mobile phase: 0.1 mol/L sodium nitrate aqueous solution, standard: polyethylene glycol, detection: differential refractive index detector 1260 RID, calculation of weight average molecular weight: molecular weight calculation program (Agilent Cirrus GPC Software), flow rate: 1.0 mL/min, column temperature: 30°C, injection volume: 20 uL, test time: 25 min.

The obtained polymer had a number average molecular weight of 25,884, a weight average molecular weight of 45,702, and a molecular weight distribution of 1.77.

Step 2) preparation of a polymer solution

The obtained polymer was dissolved in anhydrous ethanol to prepare 5 wt% P(GMA-MPC) solution.

Example 2. Coating a silane layer on the surface of a substrate

Titanium-nickel alloy products were ultrasonically washed with ethanol and deionized water, followed by immersion in diluted 35% nitric acid and hydrogen peroxide, and then washed with deionized water. They were subsequently treated by immersion in an aqueous solution of 99.5% ethanol containing 20 wt% 3-aminopropyltriethoxysilane, rinsed with deionized water, and then cured by drying at 80°C for 20-40 minutes.

Example 3. Coating polymer layer P(GMA-MPC) on the surface of a substrate

The metal treated in Example 2 was immersed in the P(GMA-MPC) solution prepared in Example 1 for 5 to 20 minutes, and cured at 80°C for 20 minutes to 1 hour. Thereafter, the metal surface was washed with anhydrous ethanol during ultrasonication for 10 minutes to eliminate non-covalently bonded polymers. The washed metal surface was dried at a temperature of about 80°C for 20 to 40 minutes.

Example 4. Coating polymer layer P(GMA-cysGMA-MPC) on the surface of a substrate

The metal treated in Example 2 was immersed in the P(GMA-MPC) solution prepared in Example 1 for 5 to 20 minutes, followed by immersion in a pH 7.2 PBS solution containing 0.1%-2% cysteine (cys) for 16 hours or more, and then cured at 80°C for 20 minutes to 1 hour. Thereafter, the metal surface was washed with ethanol during ultrasonication to eliminate non-covalently bonded polymers. The washed metal surface was dried at a temperature of about 80°C for 20 to 40 minutes.

Example 5. Preparation of a P(GMA-MPC) solution

Step 1) synthesis of a polymer

37.5 g of MPC (purchased from TCI, lot no. KDW4G-PB) and 12.1 g of GMA (purchased from Adamas, lot no. P1909690) were dissolved in 2000 ml of anhydrous ethanol, placed in a three-necked flask equipped with a thermometer and a condenser, and purged with nitrogen for 30 min. Thereafter, 1.2 g of azobisisobutyronitrile (purchased from Adamas, lot no. P1871519) was added at 60°C, and a polymerisation reaction was carried out for 10 to 20 hours. After the polymerisation, a polymer was precipitated in tetrahydrofuran and recovered as a white powder by vacuum drying at 40°C. $^1$H NMR analysis confirmed that the obtained polymer contained 52 mol% MPC.

$^1$H-NMR (400MHz, $CD_3OD$): δ 4.35, $-COOCH_2CH-$; δ 4.24, $-COOCH_2CH_2O-$; δ 4.11, $-OCH_2CH_2N(CH_3)_3$; δ 3.76, $-OCH_2CH_2N(CH_3)_3$; δ 3.33, $-CH_2N(CH_3)_3$; δ 3.14, $-CHOCH_2$; δ 2.91 and δ 2.74, $-CHOCH_2$; δ 2.00 and δ 1.92, $-CH_2-C$.

Determination of the molecular weight of the polymer

2 mg of the obtained polymer was dissolved in 1 g of 0.1 mol/L sodium nitrate aqueous solution.

Test conditions: column: Shodex (SB-806M HQ), mobile phase: 0.1 mol/L sodium nitrate aqueous solution, standard: polyethylene glycol, detection: differential refractive index detector 1260 RID, calculation of weight average molecular weight: molecular weight calculation program (Agilent Cirrus GPC Software), flow rate: 1.0 mL/min, column temperature: 30°C, injection volume: 20 uL, test time: 25 min.

The obtained polymer had a number average molecular weight of 18,277, a weight average molecular weight of 37,760, and a molecular weight distribution of 2.07.

Step 2) preparation of a polymer solution

The obtained polymer was dissolved in ethanol to prepare 5 wt% P(GMA-MPC) solution.

Example 6. Coating polymer layer P(GMA-MPC) on the surface of a substrate

The metal treated in Example 2 was immersed in the P(GMA-MPC) solution prepared in Example 5 for 5 to 20 minutes, and cured at 80°C for 20 minutes to 1 hour. Thereafter, the metal surface was washed with ethanol during ultrasonication for 10 minutes to eliminate non-covalently bonded polymers. The metal was then immersed in the P(GMA-MPC) solution prepared in Example 5 for 5 to 20 minutes, and cured at 80°C for 20 minutes to 1 hour. Thereafter, the metal surface was washed with ethanol during ultrasonication for 10 minutes to eliminate non-covalently bonded polymers. Finally, the washed metal surface was dried at a temperature of about 80°C for 20 to 40 minutes.

Example 7. Coating polymer layer P(GMA-cysGMA-MPC) on the surface of a substrate

The metal treated in Example 2 was immersed in the P(GMA-MPC) solution prepared in Example 5 for 5 to 20 minutes, and cured at 80°C for 20 minutes to 1 hour. Thereafter, the metal surface was washed with ethanol during ultrasonication for 10 minutes to eliminate non-covalently bonded polymers. The metal was then immersed in the P(GMA-MPC) solution prepared in Example 5 for 5 to 20 minutes, and cured at 80°C for 20 minutes to 1 hour. Thereafter, the metal surface was washed with ethanol during ultrasonication for 10 minutes to eliminate non-covalently bonded polymers.

The treated metal was then immersed in a pH 7.2 PBS solution containing 0.1%-2% cysteine (cys) for 16 hours or more. Thereafter, the metal surface was washed with ethanol during ultrasonication to eliminate non-covalently bonded polymers. The washed metal surface was dried at a temperature of about 80°C for 20 to 40 minutes.

Example 8. Coating polymer layer P(GMA-glyGMA-MPC) on the surface of a substrate

The metal treated in Example 2 was immersed in the P(GMA-MPC) solution prepared in Example 5 for 5 to 20 minutes, and cured at 80°C for 20 minutes to 1 hour. Thereafter, the metal surface was washed with ethanol during ultrasonication for 10 minutes to eliminate non-covalently bonded polymers. The metal was then immersed in the P(GMA-MPC) solution prepared in Example 5 for 5 to 20 minutes, and cured at 80°C for 20 minutes to 1 hour. Thereafter, the metal surface was washed with ethanol during ultrasonication for 10 minutes to eliminate non-covalently bonded polymers.

The treated metal was then immersed in a pH 7.2 PBS solution containing 0.1%-2% glycine (gly) for 16 hours or more. Thereafter, the metal surface was washed with ethanol during ultrasonication to eliminate non-covalently bonded polymers. The washed metal surface was dried at a temperature of about 80°C for 20 to 40 minutes.

Example 9. Coating polymer layer P(GMA-serGMA-MPC) on the surface of a substrate

The metal treated in Example 2 was immersed in the P(GMA-MPC) solution prepared in Example 5 for 5 to 20 minutes, and cured at 80°C for 20 minutes to 1 hour. Thereafter, the metal surface was washed with ethanol during ultrasonication for 10 minutes to eliminate non-covalently bonded polymers. The metal was then immersed in the P(GMA-MPC) solution prepared in Example 5 for 5 to 20 minutes, and cured at 80°C for 20 minutes to 1 hour. Thereafter, the metal surface was washed with ethanol during ultrasonication for 10 minutes to eliminate non-covalently bonded polymers.

The treated metal was then immersed in a pH 7.2 PBS solution containing 0.1%-2% serine (ser) for 16 hours or more. Thereafter, the metal surface was washed with ethanol during ultrasonication to eliminate non-covalently bonded polymers. The washed metal surface was dried at a temperature of about 80°C for 20 to 40 minutes.

Example 10. Coating polymer layer P(GMA-gluGMA-MPC) on the surface of a substrate

The metal treated in Example 2 was immersed in the P(GMA-MPC) solution prepared in Example 5 for 5 to 20 minutes, and cured at 80°C for 20 minutes to 1 hour. Thereafter, the metal surface was washed with ethanol during ultrasonication for 10 minutes to eliminate non-covalently bonded polymers. The metal was then immersed in the P(GMA-MPC) solution prepared in Example 5 for 5 to 20 minutes, and cured at 80°C for 20 minutes to 1 hour. Thereafter, the metal surface was washed with ethanol during ultrasonication for 10 minutes to eliminate non-covalently bonded polymers.

The treated metal was then immersed in a pH 7.2 PBS solution containing 0.1%-2% glutamic acid (glu) for 16 hours or more. Thereafter, the metal surface was washed with ethanol during ultrasonication to eliminate non-covalently bonded polymers. The washed metal surface was dried at a temperature of about 80°C for 20 to 40 minutes.

Example 11. Coating polymer layer P(GMA-lysGMA-MPC) on the surface of a substrate

The metal treated in Example 2 was immersed in the P(GMA-MPC) solution prepared in Example 5 for 5 to 20 minutes, and cured at 80°C for 20 minutes to 1 hour. Thereafter, the metal surface was washed with ethanol during ultrasonication for 10 minutes to eliminate non-covalently bonded polymers. The metal was then immersed in the P(GMA-MPC) solution prepared in Example 5 for 5 to 20 minutes, and cured at 80°C for 20 minutes to 1 hour. Thereafter, the metal surface was washed with ethanol during ultrasonication for 10 minutes to eliminate non-covalently bonded polymers.

The treated metal was then immersed in a pH 7.2 PBS solution containing 0.1%-2% lysine (lys) for 16 hours or more. Thereafter, the metal surface was washed with ethanol during ultrasonication to eliminate non-covalently bonded polymers. The washed metal surface was dried at a temperature of about 80°C for 20 to 40 minutes.

Example 12. Preparation of a P(GMA-MPC) solution

Step 1) synthesis of a polymer

1.0 g of MPC (purchased from TCI, lot no. KDW4G-PB) and 0.72 g of GMA (purchased from Adamas, lot no. P1909690) were dissolved in 30.0 ml of anhydrous ethanol, placed in a three-necked flask equipped with a thermometer and a condenser, and purged with nitrogen for 30 min. Thereafter, 0.05 g of azobisisobutyronitrile (purchased from Adamas, lot no. P1871519) was added at 60°C, and a polymerisation reaction was carried out for 10 to 20 hours. After the

polymerisation, a polymer was precipitated in tetrahydrofuran and recovered as a white powder by vacuum drying at 40°C. $^1H$ NMR analysis confirmed that the obtained polymer contained 42 mol% MPC.

$^1H$-NMR (400MHz, $CD_3OD$): δ 4.35, $-COOCH_2CH-$; δ 4.24, $-COOCH_2CH_2O-$; δ 4.11, $-OCH_2CH_2N(CH_3)_3$; δ 3.76, $-OCH_2CH_2N(CH_3)_3$; δ 3.33, $-CH_2N(CH_3)_3$; δ 3.14, $-CHOCH_2$; δ 2.91 and δ 2.74, $-CHOCH_2$; δ 2.00 and δ 1.92, $-CH_2-C$.

Determination of the molecular weight of the polymer

2 mg of the obtained polymer was dissolved in 1 g of 0.1 mol/L sodium nitrate aqueous solution.

Test conditions: column: Shodex (SB-806M HQ), mobile phase: 0.1 mol/L sodium nitrate aqueous solution, standard: polyethylene glycol, detection: differential refractive index detector 1260 RID, calculation of weight average molecular weight: molecular weight calculation program (Agilent Cirrus GPC Software), flow rate: 1.0 mL/min, column temperature: 30°C, injection volume: 20 uL, test time: 25 min.

The obtained polymer had a number average molecular weight of 10,665, a weight average molecular weight of 23,912, and a molecular weight distribution of 2.24.

Step 2) preparation of a polymer solution

The obtained polymer was dissolved in ethanol to prepare 5 wt% P(GMA-MPC) solution.

Example 13. Coating polymer layer P(GMA-MPC) on the surface of a substrate

The metal treated in Example 2 was immersed in the P(GMA-MPC) solution prepared in Example 12 for 5 to 20 minutes, and cured at 80°C for 20 minutes to 1 hour. Thereafter, the metal surface was washed with ethanol during ultrasonication for 10 minutes to eliminate non-covalently bonded polymers. The metal was then immersed in the P(GMA-MPC) solution prepared in Example 12 for 5 to 20 minutes, and cured at 80°C for 20 minutes to 1 hour. Thereafter, the metal surface was washed with ethanol during ultrasonication for 10 minutes to eliminate non-covalently bonded polymers. Finally, the washed metal surface was dried at a temperature of about 80°C for 20 to 40 minutes.

Example 14. Coating polymer layer P(GMA-cysGMA-MPC) on the surface of a substrate

The metal treated in Example 2 was immersed in the P(GMA-MPC) solution prepared in Example 12 for 5 to 20 minutes, and cured at 80°C for 20 minutes to 1 hour. Thereafter, the metal surface was washed with ethanol during ultrasonication for 10 minutes to eliminate non-covalently bonded polymers. The metal was then immersed in the P(GMA-MPC) solution prepared in Example 12 for 5 to 20 minutes, and cured at 80°C for 20 minutes to 1 hour. Thereafter, the metal surface was washed with ethanol during ultrasonication for 10 minutes to eliminate non-covalently bonded polymers.

The treated metal was then immersed in a pH 7.2 PBS solution containing 0.1%-2% cysteine (cys) for 16 hours or more. Thereafter, the metal surface was washed with ethanol during ultrasonication to eliminate non-covalently bonded polymers. The washed metal surface was dried at a temperature of about 80°C for 20 to 40 minutes.

Biological evaluation

The present disclosure is further described and explained below in conjunction with test examples, but these examples are not intended to limit the scope of the present disclosure.

Test example 1. Cytotoxicity test

The cytotoxicity test was conducted with reference to the *in vitro* cell test procedure (MTT) assay 2.0, and was evaluated based on the cell viability.

1. Instruments and reagents

| Reagent name | Manufacturer | Catalogue no./Lot no. |
|---|---|---|
| Foetal bovine serum 1 | Thermo Fisher Scientific Co., Ltd. | 10099-141C |
| Antibiotic 1 | Thermo Fisher Scientific Co., Ltd. | 15140-122 |
| Foetal bovine serum 2 | Sijiqing Company | 21090704 |
| Antibiotic 2 | Gibco | 2441834 |

(continued)

| Reagent name | Manufacturer | Catalogue no./Lot no. |
|---|---|---|
| MEM | Thermo Fisher Scientific Co., Ltd. | C12571500BT |
| MTT | Sigma-Aldrich Shanghai Trading Co., Ltd. | M2128-1G |
| L929 cells | Centre for Excellence in Molecular Cell Science, Chinese Academy of Sciences | Primary cells |
| Equipment name | Manufacturer | Model |
| Pipette | Eppendorf Life Sciences | 100-1000 μl |
| Cell counter | Shanghai Ruiyu Biotechnology Co., Ltd. | IC1000 |
| Microplate shaker | Titan | MS-4 |
| Microplate reader | Thermo Fisher Scientific Co., Ltd. | Muitiskan FC |

2. Experimental method

1) Preparation of a MTT solution

MTT powder was dissolved in phenol red-free and additive-free MEM medium at a concentration of 1 mg/mL, and sterilized by filtration through a 0.22 μm microporous filter membrane.

2) Preparation of a culture medium

L929 cells were cultured in MEM medium containing 10% foetal bovine serum 1 and antibiotic 1 (100 U/ml penicillin and 100 μg/ml streptomycin) in a 37°C, 5% $CO_2$ incubator. When the cells grew to approximately 80% confluence, they were pre-washed with a PBS solution. After removing the pre-wash solution, the cells were digested with 0.25% trypsin (containing EDTA). Once the cells retracted and became rounded with enlarged intercellular spaces, complete medium (89% MEM medium + 10% foetal bovine serum 2 + 1% antibiotic 2) was added to terminate digestion. The cells were gently pipetted to detach them, and the resulting cell suspension was transferred to a centrifuge tube and centrifuged (1000 rpm, 3 min). The supernatant was discarded, the cell pellet was added to the complete medium to prepare a single-cell suspension, and the cells were redispersed in the culture medium for later use.

3) Cell inoculation

10 uL of the single-cell suspension was thoroughly mixed with 10 μL of 0.25% trypan blue stain (configured at a 1 : 1 ratio) in a centrifuge tube. Subsequently, 10 μL of the mixture was added to the cell counting plate using a pipette and counted using a cell counter. The cell density was calculated according to the formula: "number of cells in single-cell suspension/ml = cell count result $\times$ 2", and the single-cell suspension was adjusted with complete medium to a cell concentration of $1.0 \times 10^6$ cells/ml for later use.

4) Preparation of extracts

Test samples: metal products containing polymer coatings obtained from Example 3 and Example 4.

Blank control: MEM medium containing 10% foetal bovine serum (no sample added).

Negative control: high-density polyethylene (extracted at a ratio of 0.2 g of negative control to 1 mL of cell culture medium); solvent: MEM medium containing 10% foetal bovine serum.

Positive control: 20% DMSO solution; solvent: MEM medium containing 10% foetal bovine serum.

The test samples were placed in sterile centrifuge tubes, and MEM medium containing 10% foetal bovine serum was added. These tubes were subsequently subjected to extraction by incubation in an air-bath shaker at 37°C with a shaking frequency of 60 rpm for 24 h.

Blank, negative, and positive controls were established and subjected to the same extraction conditions and duration as the test samples.

5) Cell culture

The extract was added to a 96-well plate and cultured for 24 h. The cell status and quantity were observed under

an inverted microscope to confirm that there was no obvious microscopic difference between the cells in each well.

The original culture medium was aspirated. Then, 100 μL of the test sample extract, negative control solution, positive control solution, and blank control solution were added to 6 wells per column (excluding the peripheral wells), respectively. The plate was subsequently incubated in a cell culture incubator (37°C, 5% $CO_2$) for 24 h.

6) MTT assay

After culturing for 24 h, the 96-well plate was removed from the $CO_2$ incubator, and the cell morphology and quantity were observed under a microscope to make a preliminary qualitative judgment on the test results. Abnormalities such as massive cell detachment or abnormal cell status were recorded to correct the final data or to discard the test results.

50 uL of 1 mg/ml MTT solution was added to each well using a pipette, and the plate was returned to the $CO_2$ incubator for another 2 h. The supernatant was aspirated and discarded, 100 μL of isopropanol solution was added to each well, and the plate was shaken on a microplate shaker for 10 min or until the formazan crystals were completely dissolved. The bottom surface of the 96-well plate was wiped clean with a dust-free cloth. The absorbance was measured using a microplate reader with a primary absorption wavelength of 570 nm and a reference wavelength of 630 nm. The cell viability (Viab.%) was calculated according to the following formula:

$$Viab.\% = \frac{100 \times OD_{570e}}{OD_{570b}}$$

where OD570e is the average absorbance of the test sample (100% extract) or the positive/negative control, and OD570b is the average absorbance of the blank control.

3. Experimental results

Table 1. Cell viability

| Test sample | Example 3 | Example 4 |
|---|---|---|
| Cell viability (%) | 73% | 108% |

Test example 2. Testing the coagulation performance of metal products containing different polymer coatings using the platelet adhesion method

1. Instruments and reagents

| Reagent name | Manufacturer | Catalogue no. |
|---|---|---|
| PBS buffer | Adamas life | C8020-500 mL |
| Glutaraldehyde solution | Adamas | 14376C |
| Equipment name | Manufacturer | Model |
| Centrifuge | BeckMan | Allegra 64R |
| 24-well plate | WHB | WHB-24-ZX |

2. Experimental animals

| Animal | Body weight | Gender | Source | Quality inspection unit |
|---|---|---|---|---|
| New Zealand rabbit (conventional grade) | 2.0 Kg | Male | Huimin Street, Jiashan County Shengwang Family Farm | Hangzhou Medical College |

3. Experimental method

The rabbit whole blood was centrifuged in a centrifuge for 10 min, and the supernatant was collected into a centrifuge tube; centrifugation was performed again for 10 min, and the upper half of the plasma in the centrifuge tube was discarded to obtain rabbit platelet-rich plasma (PRP). The sample surface was gently rinsed with PBS buffer and placed in a 24-well microplate, fresh PRP was added to immerse the metal products, and the plate was incubated at 37°C in a constant-temperature incubator for 2 hours. After incubation, the metal products were washed three times with PBS buffer, 2.5% glutaraldehyde solution was added to fix the platelets on the surface of the metal products, and the products were placed in a refrigerator for 24 hours to complete fixation. The fixed samples were washed three times with PBS buffer and immersed in 20%, 40%, 60%, 80%, and 100% ethanol solutions for 10 minutes in sequence.

After the metal products were dehydrated, they were dried at room temperature, and the platelet adhesion and activation on the surface of the metal products were observed using a scanning electron microscope. The experimental results are as shown in the following table:

Table 2. Anticoagulation experimental results

| Metal product | Characteristics | Platelet count/mm$^2$ |
|---|---|---|
| Titaniumnickel alloy | Abundant platelets, with pseudopodia, in an activated state. | 35700 |
| Example 3 | Abundant platelets, unevenly distributed, no pseudopodia observed. Some darker contrast areas are devoid of platelets. | 6660-8760 |
| Example 4 | Sparse platelets, no pseudopodia, in a non-activated state. | 400-1880 |

Test example 3. Testing the coagulation performance of metal products containing different polymer coatings using the platelet adhesion method

1. Instruments and reagents

| Reagent name | Manufacturer | Catalogue no. |
|---|---|---|
| PBS buffer | Adamas life | C8020-500 mL |
| Glutaraldehyde solution | Adamas | 14376C |
| Equipment name | Manufacturer | Model |
| Centrifuge | BeckMan | Allegra 64R |
| 24-well plate | WHB | WHB-24-ZX |

2. Experimental animals

| Animal | Body weight | Gender | Source | Quality inspection unit |
|---|---|---|---|---|
| New Zealand rabbit (conventional grade) | 2.0 Kg | Male | Huimin Street, Jiashan County Shengwang Family Farm | Hangzhou Medical College |

3. Experimental method

The rabbit whole blood was centrifuged in a centrifuge for 10 min, and the supernatant was collected into a centrifuge tube; centrifugation was performed again for 10 min, and the upper half of the plasma in the centrifuge tube was discarded to obtain rabbit platelet-rich plasma (PRP). The sample surface was gently rinsed with PBS buffer and placed in a 24-well microplate, fresh PRP was added to immerse the metal products, and the plate was incubated at 37°C in a constant-temperature incubator for 2 hours. After incubation, the metal products were washed three times with PBS buffer, 2.5% glutaraldehyde solution was added to fix the platelets on the surface of the metal products, and the products were placed in a refrigerator for 24 hours to complete fixation. The fixed samples were washed three times with PBS buffer and immersed in 20%, 40%, 60%, 80%, and 100% ethanol solutions for 10 minutes in sequence.

After the metal products were dehydrated, they were dried at room temperature, and the platelet adhesion and activation on the surface of the metal products were observed using a scanning electron microscope. The experimental results are as shown in the following table:

Table 3. Anticoagulation experimental results

| Metal product | Characteristics | Platelet count/$mm^2$ |
|---|---|---|
| Titaniumnickel alloy | Abundant platelets, with pseudopodia, in an activated state. | 10370 |
| Example 6 | Abundant platelets, unevenly distributed, no pseudopodia observed. Some darker contrast areas are devoid of platelets. | 7009 |
| Example 7 | Sparse platelets, no pseudopodia, in a non-activated state. | 4202-4626 |
| Example 8 | | 3858-4524 |
| Example 10 | | 3596-4666 |
| Example 11 | | 4100-5333 |

Test example 4. Testing the coagulation performance of metal products containing different polymer coatings using the platelet adhesion method

1. Instruments and reagents

| Reagent name | Manufacturer | Catalogue no. |
|---|---|---|
| PBS buffer | Adamas life | C8020-500 mL |
| Glutaraldehyde solution | Adamas | 14376C |
| Equipment name | Manufacturer | Model |
| Centrifuge | BeckMan | Allegra 64R |
| 24-well plate | WHB | WHB-24-ZX |

2. Experimental animals

| Animal | Body weight | Gender | Source | Quality inspection unit |
|---|---|---|---|---|
| New Zealand rabbit (conventional grade) | 2.0 Kg | Male | Huimin Street, Jiashan County Shengwang Family Farm | Hangzhou Medical College |

3. Experimental method

The rabbit whole blood was centrifuged in a centrifuge for 10 min, and the supernatant was collected into a centrifuge tube; centrifugation was performed again for 10 min, and the upper half of the plasma in the centrifuge tube was

discarded to obtain rabbit platelet-rich plasma (PRP). The sample surface was gently rinsed with PBS buffer and placed in a 24-well microplate, fresh PRP was added to immerse the metal products, and the plate was incubated at 37°C in a constant-temperature incubator for 2 hours. After incubation, the metal products were washed three times with PBS buffer, 2.5% glutaraldehyde solution was added to fix the platelets on the surface of the metal products, and the products were placed in a refrigerator for 24 hours to complete fixation. The fixed samples were washed three times with PBS buffer and immersed in 20%, 40%, 60%, 80%, and 100% ethanol solutions for 10 minutes in sequence.

After the metal products were dehydrated, they were dried at room temperature, and the platelet adhesion and activation on the surface of the metal products were observed using a scanning electron microscope. The experimental results are as shown in the following table:

Table 4. Anticoagulation experimental results

| Metal product | Characteristics | Platelet count/$mm^2$ |
|---|---|---|
| Titanium-nickel alloy | Abundant platelets, with pseudopodia, in an activated state. | 14909 |
| Example 13 | Abundant platelets, unevenly distributed, no pseudopodia observed. Some darker contrast areas are devoid of platelets. | 4250 |
| Example 14 | Sparse platelets, no pseudopodia, in a non-activated state. | 3111 |

**Claims**

**1.** A material, comprising a substrate and a coating, wherein the coating comprises a polymer, and the polymer comprises a structural unit having a group represented by formula 1-a, a structural unit having a group represented by formula 1-b, and a structural unit having a group represented by formula 1-c,

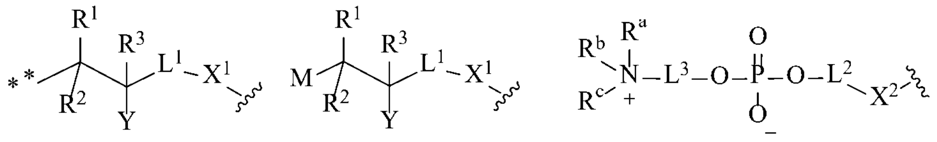

1-a , 1-b and 1-c ,

wherein:

** represents an end connected to the surface of the substrate, and the surface of the substrate is optionally treated with a modifier;

$X^1$ and $X^2$ are the same or different, and are each independently selected from -O-, -S- or -N($R^d$)-;

Y is selected from -OH, -SH or -N($R^eR^f$);

$R^1$, $R^2$ and $R^3$ are the same or different, and are each independently selected from hydrogen, halogen, hydroxyl, mercapto, nitro, carboxyl, amino, cyano, $C_{1-6}$ alkyl or $C_{1-6}$ alkoxy, and the $C_{1-6}$ alkyl and $C_{1-6}$ alkoxy are optionally substituted with one or more groups selected from halogen, hydroxyl, mercapto, nitro, carboxyl, amino, cyano, $C_{1-6}$ alkyl and $C_{1-6}$ alkoxy,

alternatively, $R^1$ and $R^2$, together with the atom to which they are connected, form an oxo group, a thio group, $C_{3-10}$ cycloalkyl, or 3- to 10-membered heterocycloalkyl;

$L^1$ and $L^3$ are the same or different, and are each independently selected from a bond, alkylene or heteroalkylene, and the alkylene and heteroalkylene are optionally substituted with one or more groups selected from $C_{1-6}$ alkyl, $C_{1-6}$ haloalkyl, $C_{1-6}$ hydroxyalkyl, $C_{1-6}$ aminoalkyl, $C_{1-6}$ alkoxy, $C_{1-6}$ haloalkoxy, $C_{1-6}$ hydroxyalkoxy, $C_{1-6}$ aminoalkoxy, halogen, amino, hydroxyl, nitro, cyano, mercapto, carboxyl, an oxo group, a thio group or sulphonyl;

$L^2$ is selected from alkylene or heteroalkylene, and the alkylene and heteroalkylene are optionally substituted with one or more groups selected from $C_{1-6}$ alkyl, $C_{1-6}$ haloalkyl, $C_{1-6}$ hydroxyalkyl, $C_{1-6}$ aminoalkyl, $C_{1-6}$ alkoxy, $C_{1-6}$ haloalkoxy, $C_{1-6}$ hydroxyalkoxy, $C_{1-6}$ aminoalkoxy, halogen, amino, hydroxyl, nitro, cyano, mercapto, carboxyl, an oxo group, a thio group or sulphonyl;

M is a residue of amino acid (A), and the M is optionally substituted with one or more groups selected from halogen, amino, hydroxyl, nitro, cyano, amino, mercapto, carboxyl, $C_{1-6}$ alkyl or $C_{1-6}$ alkoxy;

$R^a$, $R^b$, $R^c$, $R^d$, $R^e$ and $R^f$ are the same or different, and are each independently selected from hydrogen, halogen, hydroxyl, $C_{1-6}$ alkyl or $C_{1-6}$ alkoxy, and the $C_{1-6}$ alkyl or $C_{1-6}$ alkoxy is optionally substituted with one or more

groups selected from halogen, hydroxyl, cyano, nitro, $C_{1-6}$ alkyl, $C_{1-6}$ alkoxy and $C_{3-7}$ cycloalkyl.

**2.** The material according to claim 1, wherein the polymer comprises a structural unit represented by formula I-a, a structural unit represented by formula I-b, and a structural unit represented by formula I-c,

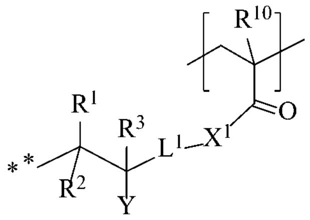

I-a

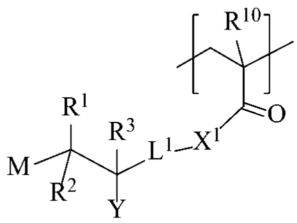

I-b

,

and

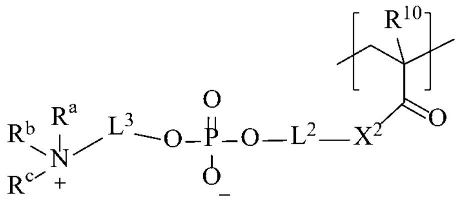

I-c

,

wherein:

** represents an end connected to the surface of the substrate;
each $R^{10}$ is independently selected from hydrogen, halogen, hydroxyl, mercapto, nitro, carboxyl, amino, cyano, $C_{1-6}$ alkyl or $C_{1-6}$ alkoxy, and the $C_{1-6}$ alkyl and $C_{1-6}$ alkoxy are optionally substituted with one or more groups selected from halogen, hydroxyl, mercapto, nitro, carboxyl, amino, cyano, $C_{1-6}$ alkyl or $C_{1-6}$ alkoxy;
$L^1$, $L^2$, $L^3$, $R^1$, $R^2$, $R^3$, M, Y, $X^1$, $X^2$, $R^a$, $R^b$, and $R^c$ are as defined in claim 1.

**3.** The material according to claim 1 or 2, wherein Y is selected from -OH or -SH, preferably -OH.

**4.** The material according to any one of claims 1 to 3, wherein $L^1$ and $L^3$ are each independently selected from a bond or $C_{1-6}$ alkylene, and the $C_{1-6}$ alkylene is optionally substituted with one or more groups selected from $C_{1-6}$ alkyl, $C_{1-6}$ haloalkyl, $C_{1-6}$ hydroxyalkyl, $C_{1-6}$ aminoalkyl, halogen, amino, hydroxyl, cyano or mercapto.

**5.** The material according to any one of claims 1 to 4, wherein $L^2$ is $C_{1-6}$ alkylene, which is optionally substituted with one or more groups selected from $C_{1-6}$ alkyl, $C_{1-6}$ haloalkyl, $C_{1-6}$ hydroxyalkyl, $C_{1-6}$ aminoalkyl, halogen, amino, hydroxyl, cyano or mercapto.

**6.** The material according to any one of claims 1 to 5, wherein $X^1$ and $X^2$ are each independently selected from -O- or -N($R^d$)-, preferably -O-, and $R^d$ is as defined in claim 1.

**7.** The material according to any one of claims 1 to 6, wherein the polymer comprises a structural unit having a group represented by formula 3-a, a structural unit having a group represented by formula 3-b, and a structural unit having a group represented by formula 3-c,

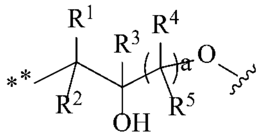
3-a ,
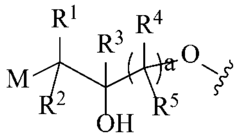
3-b and
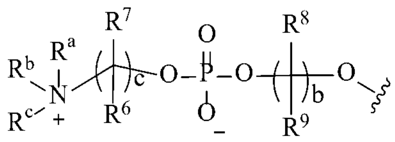
3-c ,

wherein: ** represents an end connected to the surface of the substrate;

$R^4$, $R^5$, $R^6$, $R^7$, $R^8$ and $R^9$ are the same or different, and are each independently selected from hydrogen, halogen, hydroxyl, mercapto, nitro, carboxyl, amino, cyano, $C_{1-6}$ alkyl, $C_{1-6}$ alkoxy, $C_{3-7}$ cycloalkyl or 3- to 7-membered heterocycloalkyl, and the $C_{1-6}$ alkyl, $C_{1-6}$ alkoxy, $C_{3-7}$ cycloalkyl or 3- to 7-membered heterocycloalkyl is optionally substituted with one or more groups selected from halogen, hydroxyl, mercapto, nitro, carboxyl, amino, cyano, $C_{1-6}$ alkyl or $C_{1-6}$ alkoxy,

alternatively, $R^4$ and $R^5$, $R^6$ and $R^7$, or $R^8$ and $R^9$, together with the atom to which they are connected, form an oxo group, a thio group, $C_{3-10}$ cycloalkyl, or 3- to 10-membered heterocycloalkyl;

a is selected from 0, 1, 2, 3, 4, 5, or 6;

b is selected from 1, 2, 3, 4, 5, or 6;

c is selected from 0, 1, 2, 3, 4, 5, or 6;

$R^1$, $R^2$, $R^3$, M, $R^a$, $R^b$, and $R^c$ are as defined in any one of the preceding claims.

**8.** The material according to any one of claims 1 to 7, wherein the polymer comprises a structural unit represented by formula III-a, a structural unit represented by formula III-b, and a structural unit represented by formula III-c,

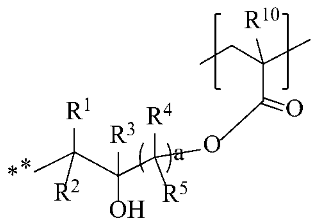
III-a ,
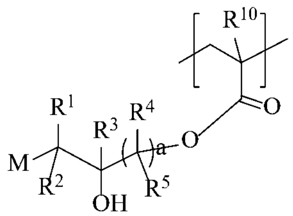
III-b

and

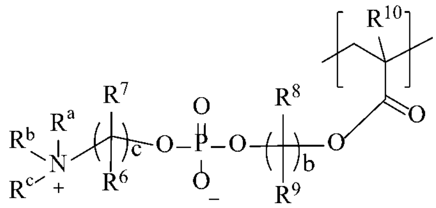
III-c ,

wherein: ** represents an end connected to the surface of the substrate;

$R^1$, $R^2$, $R^3$, $R^4$, $R^5$, $R^6$, $R^7$, $R^8$, $R^9$, $R^{10}$, M, $R^a$, $R^b$, $R^c$, a, b, and c are as defined in claim 7.

**9.** The material according to any one of claims 1 to 8, wherein the amino acid (A) is selected from glycine, alanine, valine, isoleucine, leucine, methionine, phenylalanine, tryptophan, serine, threonine, cysteine, asparagine, glutamine, tyrosine, aspartic acid, glutamic acid, lysine, arginine, histidine or taurine, preferably glycine, phenylalanine, threonine, serine, histidine or cysteine, most preferably glycine, threonine, serine or cysteine.

**10.** The material according to any one of claims 1 to 9, wherein the polymer comprises a structural unit having a group represented by formula 4-a, a structural unit having a group represented by formula 4-b, and a structural unit having a group represented by formula 4-c,

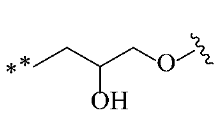
4-a
,
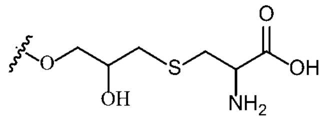
4-b
and
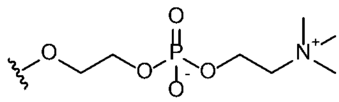
4-c
,

wherein: ** represents an end connected to the surface of the substrate.

**11.** The material according to any one of claims 1 to 10, wherein the polymer comprises a structural unit represented by formula IV-a, a structural unit represented by formula IV -b, and a structural unit represented by formula IV-c,

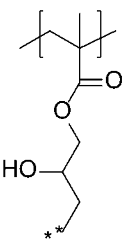
IV-a
,
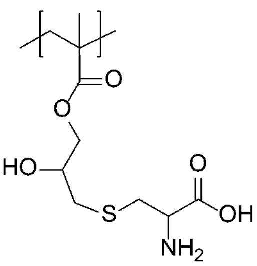
IV-b
and
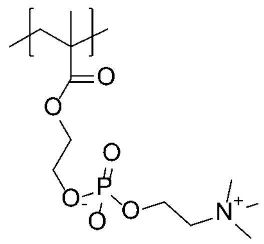
IV-c
,

wherein: ** represents an end connected to the surface of the substrate.

**12.** The material according to claim 11, wherein the polymer comprises a structural unit represented by formula IV-A, a structural unit represented by formula IV-B, and a structural unit represented by formula IV-C,

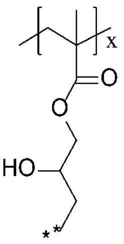
IV-A
,
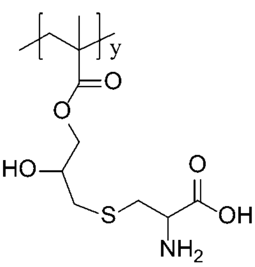
IV-B
and
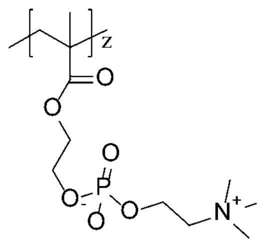
IV-C
,

wherein: ** represents an end connected to the surface of the substrate;
on a molar ratio basis, z : (x + y) is selected from 20 : 80 to 90 : 10, preferably 40 : 60, 45 : 55, 50 : 50, 55 : 45, 60 : 40, 65 : 35 or 70 : 30, most preferably 70 : 30.

**13.** The material according to any one of claims 1 to 12, wherein the modifier is a coupling agent represented by formula V,

$$(R^{11}-O)_3 Si-R^{12}$$

V
,

wherein:

$R^{11}$ is selected from $C_{1-6}$ alkyl, $C_{1-6}$ haloalkyl, $C_{1-6}$ hydroxyalkyl or $C_{1-6}$ aminoalkyl;
$R^{12}$ is selected from $C_{1-6}$ hydroxyalkyl, $C_{1-6}$ aminoalkyl, $C_{1-6}$ carboxyalkyl or $C_{1-6}$ mercaptoalkyl, and the $C_{1-6}$ hydroxyalkyl, $C_{1-6}$ aminoalkyl, $C_{1-6}$ carboxyalkyl and $C_{1-6}$ mercaptoalkyl are optionally substituted with one or more groups selected from $C_{1-6}$ hydroxyalkyl, $C_{1-6}$ aminoalkyl, $C_{1-6}$ carboxyalkyl or $C_{1-6}$ mercaptoalkyl.

**14.** The material according to claim 13, wherein the $R^{11}$ is $C_{1-6}$ alkyl, preferably methyl, ethyl, propyl or butyl, most preferably ethyl,
the coupling agent is connected to the surface of the substrate via $R^{11}$ to form a silane layer, and the surface of the substrate is optionally hydroxylated.

**15.** The material according to claim 13 or 14, wherein the $R^{12}$ is $C_{1-6}$ aminoalkyl, and the $C_{1-6}$ aminoalkyl is optionally substituted with one or more groups selected from $C_{1-6}$ hydroxyalkyl, $C_{1-6}$ aminoalkyl, $C_{1-6}$ carboxyalkyl or $C_{1-6}$ mercaptoalkyl, preferably methylamino, ethylamino, propylamino or butylamino, most preferably propylamino,
the coupling agent is connected to the ** end of the polymer as defined in any one of claims 1 to 12 via the amino group of $R^{12}$ to form a polymer layer.

**16.** The material according to any one of claims 13 to 15, wherein the coupling agent is selected from 3-aminopropyltrimethoxysilane (KH540), 3-aminopropyltriethoxysilane (KH550), 3-(2-aminoethyl)aminopropyltrimethoxysilane (KH792), 3-(2-aminoethyl)aminopropyltriethoxysilane (KH791), 3-(2-aminoethylamino)propyldimethoxymethylsilane (KH602), 3-hydroxypropyltriethoxysilane, 3-hydroxypropyltrimethoxysilane, 3-mercaptopropyltrimethoxysilane, 3-mercaptopropyltriethoxysilane, 4-(trimethoxysilyl)butyric acid, 4-(triethoxysilyl)butyric acid, bis(2-hydroxyethyl)aminopropyltriethoxysilane, bis(2-hydroxyethyl)aminopropyltrimethoxysilane, or 3-aminopropyltriisopropoxysilane, preferably 3-aminopropyltrimethoxysilane (KH540) or 3-aminopropyltriethoxysilane (KH550), most preferably 3-aminopropyltriethoxysilane (KH550).

**17.** A material, comprising a substrate and a coating, wherein the coating is formed by reacting a polymer comprising a structural unit having a group represented by formula 1-d and a structural unit having a group represented by formula 1-c with the coupling agent as defined in any one of claims 13 to 16, and then reacting with the amino acid (A) as defined in claim 9,

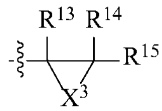

1-d

,

wherein:

$X^3$ is selected from -O-, -S- or $-N(R^d)-$;
$R^{13}$, $R^{14}$ and $R^{15}$ are the same or different, and are each independently selected from hydrogen, halogen, hydroxyl, mercapto, nitro, carboxyl, amino, cyano, $C_{1-6}$ alkyl or $C_{1-6}$ alkoxy, and the $C_{1-6}$ alkyl and $C_{1-6}$ alkoxy are optionally substituted with one or more groups selected from halogen, hydroxyl, mercapto, nitro, carboxyl, amino, cyano, $C_{1-6}$ alkyl or $C_{1-6}$ alkoxy,
alternatively, $R^{14}$ and $R^{15}$, together with the atom to which they are connected, form an oxo group, a thio group, $C_{3-10}$ cycloalkyl, or 3- to 10-membered heterocycloalkyl;
formula 1-c and $R^d$ are as defined in claim 1.

**18.** The material according to claim 17, wherein the polymer comprises a structural unit represented by formula I-d and a structural unit represented by formula I-c,

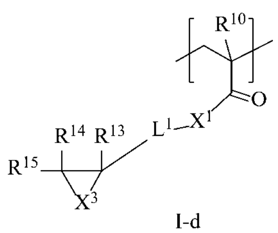

I-d

,

wherein: formula I -c, $R^{10}$, $L^1$, $X^1$, $X^3$, $R^{13}$, $R^{14}$, and $R^{15}$ are as defined in claim 17.

**19.** The material according to claim 17 or 18, wherein the $X^3$ is selected from -O- or -S-, preferably -O-.

**20.** The material according to any one of claims 17 to 19, wherein the polymer comprises a structural unit represented by formula IV-c and a structural unit represented by formula IV-d,

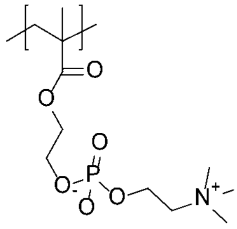

IV-c

and

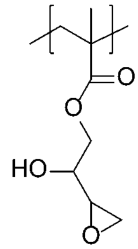

IV-d

.

**21.** The material according to claim 20, wherein the polymer comprises a structural unit represented by formula IV-C' and a structural unit represented by formula IV-D,

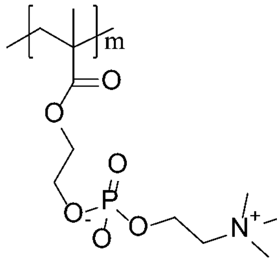

IV-C'

and

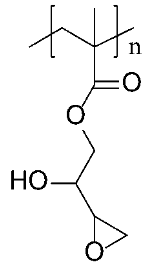

IV-D

,

wherein, on a molar ratio basis, m : n is selected from 20 : 80 to 90 : 10, preferably 40 : 60, 45 : 55, 50 : 50, 55 : 45, 60 : 40, 65 : 35 or 70 : 30, most preferably 70 : 30.

**22.** A coating, comprising a polymer, wherein the polymer comprises the polymer as defined in any one of claims 1 to 21.

**23.** The coating according to claim 22, further comprising a coupling agent represented by formula V

$$(R^{11}-O)_3Si-R^{12}$$

V

,

wherein:

$R^{11}$ is selected from $C_{1-6}$ alkyl, $C_{1-6}$ haloalkyl, $C_{1-6}$ hydroxyalkyl or $C_{1-6}$ aminoalkyl;
$R^{12}$ is selected from $C_{1-6}$ hydroxyalkyl, $C_{1-6}$ aminoalkyl, $C_{1-6}$ carboxyalkyl or $C_{1-6}$ mercaptoalkyl, and the $C_{1-6}$ hydroxyalkyl, $C_{1-6}$ aminoalkyl, $C_{1-6}$ carboxyalkyl and $C_{1-6}$ mercaptoalkyl are optionally substituted with one or more groups selected from $C_{1-6}$ hydroxyalkyl, $C_{1-6}$ aminoalkyl, $C_{1-6}$ carboxyalkyl or $C_{1-6}$ mercaptoalkyl.

**24.** A method for preparing the coating according to claim 22 or 23, comprising : reacting a polymer comprising a structural unit having a group represented by formula 1-d and a structural unit having a group represented by formula 1-c with the coupling agent as defined in any one of claims 13 to 16, and then reacting with amino acid (A), wherein
the amino acid (A) is selected from glycine, alanine, valine, isoleucine, leucine, methionine, phenylalanine, tryptophan, serine, threonine, cysteine, asparagine, glutamine, tyrosine, aspartic acid, glutamic acid, lysine, arginine, histidine or taurine, preferably glycine, phenylalanine, threonine, serine, histidine or cysteine, most preferably glycine, threonine, serine or cysteine.

**25.** A method for preparing the coating according to claim 22 or 23, comprising : reacting a polymer comprising a structural unit represented by formula IV-d and a structural unit represented by formula IV-c with the coupling agent as defined in

claim 14 or 15, and then reacting with amino acid (A), wherein
the amino acid (A) is selected from glycine, alanine, valine, isoleucine, leucine, methionine, phenylalanine, tryptophan, serine, threonine, cysteine, asparagine, glutamine, tyrosine, aspartic acid, glutamic acid, lysine, arginine, histidine or taurine, preferably glycine, phenylalanine, threonine, serine, histidine or cysteine, most preferably glycine, threonine, serine or cysteine.

**26.** A method for inhibiting platelet aggregation and platelet adhesiveness caused by the exposure of blood to the surface of a material, comprising using the material according to any one of claims 1 to 21.

**27.** A method for inhibiting platelet aggregation and platelet adhesiveness caused by the exposure of blood to the surface of a material, comprising applying the coating according to claim 22 or 23 to the surface of a substrate of a material.

**28.** A method for inhibiting platelet aggregation and platelet adhesiveness caused by the exposure of blood to the surface of a material, comprising applying the coating prepared by the method according to claim 24 or 25 to the surface of a substrate of a material.

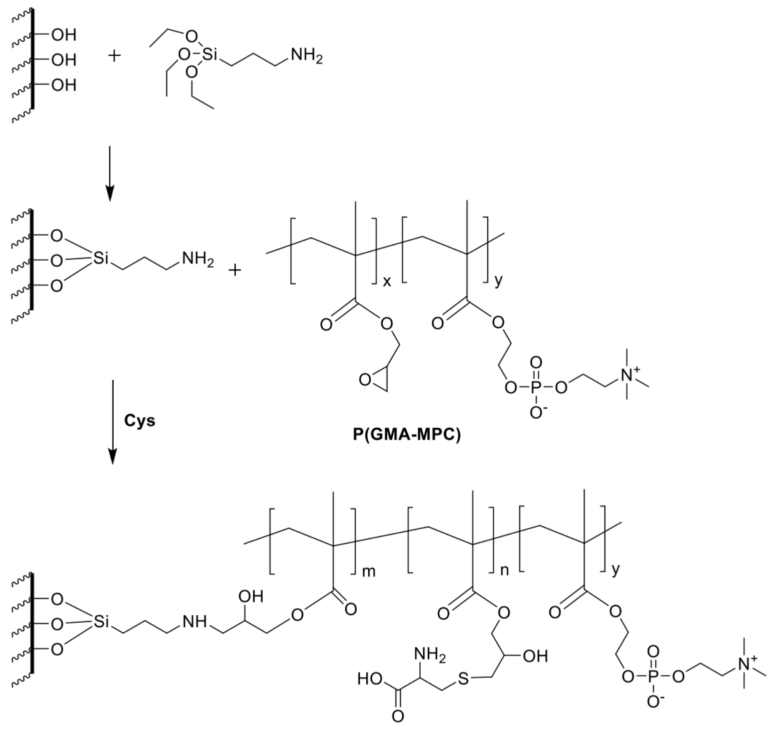
Cys
P(GMA-MPC)

*FIG. 1*

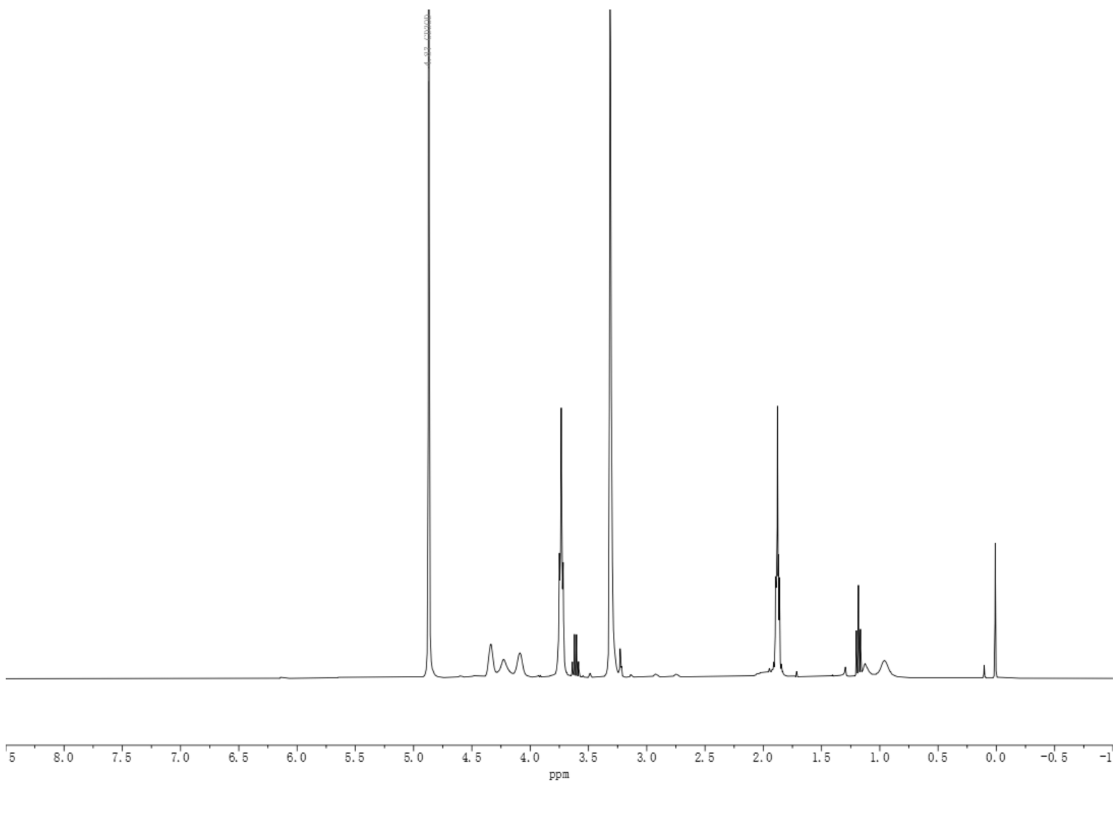
8.0
7.5
7.0
6.5
6.0
5.5
5.0
4.5
4.0
3.5
3.0
2.5
2.0
1.5
1.0
0.5
0.0
-0.5
-1
ppm

*FIG. 2*

**INTERNATIONAL SEARCH REPORT**

International application No. **PCT/CN2024/104623**

**A. CLASSIFICATION OF SUBJECT MATTER**

A61L27/34(2006.01)i; A61L27/50(2006.01)i; A61L31/14(2006.01)i; A61L31/10(2006.01)i; C08F292/00(2006.01)i

According to International Patent Classification (IPC) or to both national classification and IPC

**B. FIELDS SEARCHED**

Minimum documentation searched (classification system followed by classification symbols)

IPC: A61L27, A61L31, C08F292;

Documentation searched other than minimum documentation to the extent that such documents are included in the fields searched

Electronic data base consulted during the international search (name of data base and, where practicable, search terms used)

CNTXT, ENTXTC, VEN, WPABSC, CNKI, ISI Web of Science: 氨基酸, 氨酸, 半胱氨酸, 表面, 处理, 共聚, 聚合, 硅烷, 偶联, 基材, 基底, 两性离子, 磷酸胆碱, 磷铵, 磷酰胆碱, 缩水甘油基酯, 缩水甘油酯, 环氧, 涂层, 生物, 相容, 血, 血小板, cys, epoxide, GMA, lysine, MPC, substrate, suface, blood, phosphorylcholine, epoxy, polymer, treat, silane, coupling, amino acid, pretreat, zwitter, copolymer, platelet, cardiovascular, glycidyl, amphoteric;

**C. DOCUMENTS CONSIDERED TO BE RELEVANT**

| Category* | Citation of document, with indication, where appropriate, of the relevant passages | Relevant to claim No. |
|---|---|---|
| X | CN 109529118 A (HUANGHUAI UNIVERSITY) 29 March 2019 (2019-03-29)<br>description, paragraphs 0005-0040, and figure 1 | 1-28 |
| X | CN 107722321 A (XI'AN UNIVERSITY OF SCIENCE AND TECHNOLOGY) 23 February 2018 (2018-02-23)<br>description, paragraphs [0006]-[0025] | 1-28 |
| X | CN 115558148 A (ZHEJIANG UNIVERSITY) 03 January 2023 (2023-01-03)<br>description, paragraphs 0007-0035 and 0068-0071, and figures 1-2 | 22,27 |
| Y | CN 115558148 A (ZHEJIANG UNIVERSITY) 03 January 2023 (2023-01-03)<br>description, paragraphs 0007-0035 and 0068-0071, and figures 1-2 | 1-12, 22, 26-27 |
| X | CN 106750450 A (XI'AN UNIVERSITY OF SCIENCE AND TECHNOLOGY) 31 May 2017 (2017-05-31)<br>description, paragraphs 0007-0020 | 22,27 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **05 September 2024** | **15 September 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/104623**

**C. DOCUMENTS CONSIDERED TO BE RELEVANT**

| Category* | Citation of document, with indication, where appropriate, of the relevant passages | Relevant to claim No. |
|---|---|---|
| Y | CN 106750450 A (XI'AN UNIVERSITY OF SCIENCE AND TECHNOLOGY) 31 May 2017 (2017-05-31)<br>description, paragraphs 0007-0020 | 1-12, 22, 26-27 |
| Y | CN 108043253 A (SOUTHEAST UNIVERSITY) 18 May 2018 (2018-05-18)<br>description, paragraphs 0005-0021 | 1-12, 22, 26-27 |
| A | CN 116271231 A (LEPU MEDICAL TECHNOLOGY (BEIJING) CO., LTD.) 23 June 2023 (2023-06-23)<br>entire document | 1-28 |
| A | US 2009155335 A1 (SEMPRUS BIOSCIENCES CORP.) 18 June 2009 (2009-06-18)<br>entire document | 1-28 |
| A | JP 2018191613 A (AGC INC.) 06 December 2018 (2018-12-06)<br>entire document | 1-28 |

Form PCT/ISA/210 (second sheet) (July 2022)

**INTERNATIONAL SEARCH REPORT**
**Information on patent family members**

International application No.
**PCT/CN2024/104623**

| Patent document cited in search report | Publication date (day/month/year) | Patent family member(s) | Publication date (day/month/year) |
|---|---|---|---|
| CN 109529118 A | 29 March 2019 | None | |
| CN 107722321 A | 23 February 2018 | None | |
| CN 115558148 A | 03 January 2023 | CN 115558148 B | 23 May 2023 |
| CN 106750450 A | 31 May 2017 | CN 106750450 B | 27 October 2020 |
| CN 108043253 A | 18 May 2018 | None | |
| CN 116271231 A | 23 June 2023 | None | |
| US 2009155335 A1 | 18 June 2009 | WO 2009085096 A2 | 09 July 2009 |
| JP 2018191613 A | 06 December 2018 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2001007097 A1